(19)

Europäisches Patentamt

European Patent Office

Office européen des brevets

(11) **EP 4 438 240 A1**

(12) **EUROPEAN PATENT APPLICATION**

(43) Date of publication:
02.10.2024 Bulletin 2024/40

(21) Application number: 23165664.6

(22) Date of filing: 30.03.2023

(51) International Patent Classification (IPC):
*B25J 9/16* (2006.01)      *A61B 34/30* (2016.01)
*A61B 34/00* (2016.01)      *B25J 11/00* (2006.01)
*A61M 25/01* (2006.01)      *A61B 34/20* (2016.01)
*A61B 1/00* (2006.01)

(52) Cooperative Patent Classification (CPC):
**B25J 9/1679; A61B 34/30; A61B 34/72;**
A61B 1/00158; A61B 1/041; A61B 34/73;
A61B 2034/2065; A61M 25/0127; B25J 9/1689

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Max-Planck-Gesellschaft zur
Förderung der
Wissenschaften e.V.
80539 München (DE)**

(72) Inventors:
• **CHUNXANG, Wang
70565 Stuttgart (DE)**
• **YINGDAN, Wu
70565 Stuttgart (DE)**
• **XIAOGUANG, Dong
Nashville, TN 37212 (US)**
• **METIN, Sitti
70569 Stuttgart (DE)**

(74) Representative: **Manitz Finsterwald
Patent- und Rechtsanwaltspartnerschaft mbB
Martin-Greif-Strasse 1
80336 München (DE)**

(54) **METHOD, ROBOT, AND SYSTEM FOR PROBING ONE OR MORE PROPERTIES OF MATERIAL**

(57)    The invention relates to a method for probing one or more properties of a material (10) at a probing spot (14) at the surface (12) of the material (10) by an untethered robot (20), the robot (20) comprising a shape changeable member (30) actuatable by temporally and/or spatially variable magnetic fields. Further, the invention relates to a robot for probing one or more properties of a material (10) at a probing spot (14) at the surface (12) of the material (10), wherein the robot (20) is untethered and comprises a shape changeable member (30) actuatable by temporally and/or spatially variable magnetic fields, wherein the robot (20) comprises two footpads (22) arranged at opposite ends of the shape changeable member (30), and the robot (20) further comprises an adhesive probing patch (50) arranged along the shape changeable member (30) between the two footpads (22). Additionally, the invention relates to a system (100) for probing one or more properties of a material (10) at a probing spot (14) at the surface (12) of the material (10), comprising a magnetic actuation system (110), an imaging system (120), a data analyzing system (130), and a robot (20).

Fig. 1

**Description**

The invention relates to a method for probing one or more properties of a material at a probing spot at the surface of the material by an untethered robot, the robot comprising a shape changeable member actuatable by temporally and/or spatially variable magnetic fields. Further, the invention relates to a robot for probing one or more properties of a material at a probing spot at the surface of the material, wherein the robot is untethered and comprises a shape changeable member actuatable by temporally and/or spatially variable magnetic fields, wherein the robot comprises two footpads arranged at opposite ends of the shape changeable member, and the robot further comprises an adhesive probing patch arranged along the shape changeable member between the two footpads. Additionally, the invention relates to a system for probing one or more properties of a material at a probing spot at the surface of the material, comprising a magnetic actuation system, an imaging system, a data analyzing system, and a robot.

Information of properties of a material is often necessary throughout many modern applications, for instance for quality control during fabrication of goods and/or maintenance. Said properties can often be probed by investigating the respective material at a probing spot on the surface of the material. Measuring said properties allows identifying existing or even upcoming problems when implementing and/or using the respective material. Also, in biological system such as plants, animals or human bodies, knowing properties of a material of said systems is of advantage, for instance for research and science, but also as basic information for subsequently performed diagnostic procedures or treatments.

However, probing one or more properties of a material to obtain the above information often results in, or is even based on, at least partial destruction of the material under investigation, e.g. by removing test specimens from the material. Such methods are not applicable to materials used in devices that must not be damaged, such as containers for hazardous substances, such as toxic and/or flammable gases, or high-pressure vessels.

Also, it often happens that the properties are to be determined in places that are not directly accessible, for instance if the probing spot is located on the inside surface of a cavity. Sometimes, even a tethered probing device is not applicable, as for instance the respective cavity is or has to be kept shut with respect to the environment. Hence, for said occasions a probing device, especially a robot, which is completely remotely controllable and/or actuable, with respect to both moving and probing activities, would be of advantage. In addition, as the locations at which the probing of the respective material properties should take place and/or the environments for the during an approach of the probing device to the probing spot are often spatially limited, said probing device or robot should be as small as possible, especially smaller than 1 cm. All these limitations hold especially true for probing properties of materials within animal or human bodies, such as for instance properties of materials present at the inner surface of the gastrointestinal tract such as for instance gut tissue.

In view of the above, it is an object of the present invention to provide an improved method for probing one or more properties of a material, an improved robot for probing one or more properties of a material, and an improved system for probing one or more properties of a material which do not have the aforementioned drawbacks of the state of the art. In particular, it is an object of the present invention to provide an improved method, an improved robot, and an improved system which provide a simple, effective and especially non-invasive probing of the respective properties, without the need of a direct access to the probing spot on the surface of the material to be probed.

This object is satisfied by the respective independent patent claims. In particular, this object is satisfied by a method for probing one or more properties of a material according to independent claim 1, by robot for probing one or more properties of a material according to claim 13, and by a system for probing one or more properties of a material according to claim 16. The dependent claims describe preferred embodiments of the invention. Details and advantages described with respect to the method according to the first aspect of the invention also refer to a robot according to the second aspect of the invention and to a system according to the third aspect of the invention, and vice versa, if of technical sense.

According to a first aspect the invention the object is satisfied by a method for probing one or more properties of a material at a probing spot at the surface of the material by an untethered robot, the robot comprising a shape changeable member actuatable by temporally and/or spatially variable magnetic fields.

The method according to the present invention is characterized by the following steps:

a) Providing a temporally and/or spatially variable first magnetic field by a magnetic actuation system for actuating the shape changeable member for creating one or more types of locomotion of the robot, and guiding the robot to a probing spot of the material to be probed by accordingly altering the first magnetic field,
b) Providing a temporally and/or spatially variable second magnetic field by the magnetic actuation system for actuating the shape changeable member for creating a probing movement of the robot at the probing spot,
c) Measuring a quasi-static and/or dynamical shape change of the robot during execution of step b) by an imaging system, and
d) Determining the one or more properties of the material by a data analyzing system by analyzing the quasi-static and/or dynamical shape change measured in step c).

By implementing the method according to the present invention one or more properties of a material can be probed. In simple words, said probing is done firstly by remotely guiding a robot to a probing spot on a surface of the material to be investigated. Subsequently, the robot is, again completely remotely, actuated to perform a probing movement for creating a response of the material. Said response is measured by monitoring a shape of the robot. Finally, the measurements conducted during said monitoring are analyzed for determining the one or more properties of the material. In the following, the single steps of the method are described in more detail.

The method according to the present invention is especially intended to be carried out with an untethered robot. Said robot comprises a shape changeable member actuatable by temporally and/or spatially variable magnetic fields. The robot can comprise a single shape changeable member, a plurality of shape changeable members and/or one of more composite members consisting of several sections with different embodiments of shape changeable members.

For all entities of shape changeable members in the sense of the present invention, by applying an external magnetic field, the actual shape of the shape changeable member can be actively altered. The time and/or spatial characteristics of the applied magnetic field and the internal structure of the shape changeable member defines the reaction of the shape changeable member, in other word the shape and/or shape change of the shape changeable member. Said shape change of the shape changeable member is used for providing controlled movements of the robot, both for actually moving the robot and for probing the material, respectively. A physical connection to the robot is not necessary and hence the actuation of the robot can be provided untethered, even when the probing spot, and hence the robot, is located within a cavity without or at least with very limited connection to the ambient environment.

In the first step a) of the method according to the present invention, a temporally and/or spatially variable first magnetic field is applied by an accordingly constructed magnetic actuation system. The first magnetic field impinges onto the shape changeable member, and thereby the shape changeable member is actuated. In particular, the spatial and/or temporal characteristics of the first magnetic field are selected such that the actuation of the shape changeable member creates one or more types of locomotion of the robot, wherein different types of locomotion can be created sequentially and/or simultaneously.

In particular, during step a) of the method according to the present invention, the first magnetic field is provided such that the robot, driven by the shape changes of the shape changeable member, moves towards the probing spot. For that purpose, the first magnetic field can be altered for instance depending on, but not limited to, the position of the robot with respect to the probing spot and/or with respect to the environment in which the robot is moving and/or with respect to the type of locomotion of the robot intended to be created.

In summary, during the execution of step a) of the method according to the present invention, the robot moves towards the probing spot at which the one or more property of the material should be investigated. After execution of step a), the robot is located at said probing spot.

In the following step b) of the method according to the present invention, the magnetic field provided by the magnetic actuation system changes. In particular, the magnetic actuation system provides a second magnetic field, which again actuates the shape changeable member. For this purpose, also the second magnetic field comprises a characteristic which is spatial and/or temporal variable. However, and especially different to actuation of the shape changeable member by the first magnetic field, the second magnetic field actuates the shape changeable member such that a probing movement of the robot is carried out. In other words, the whole robot stays stationary at the probing spot, but nevertheless at least a section of the robot moves relative to the surface of the material to be probed.

In particular, said probing movement of the robot is created such that one or more parts and/or sections of the robot interact with the material. As said interaction generally depends on properties of the material, said probing movement allows probing one or more properties of the material. The above-mentioned dependencies may inter alia include a strength of the adhesion of the robot on the material and/or a size and/or phase shift of a response of the material on motions induced by the robot.

Hence, step b) provides the actual probing of the material at the probing spot. The robot carries out its probing movement and the material reacts on said probing movement according to its properties.

As described above, the material reacts to the probing movement of the robot. However, said reactions of the material, which in turn depend on the properties of the material, also influence the movement and/or shape of the robot. Consequently, also said movement and/or shape of the robot depends, at least partly, on properties of the material at the probing spot.

Hence, during step b), preferably over the whole length of the execution of step b), in an additional step c) of the method according to the present invention a quasi-static and/or dynamical shape change of the robot is measured. For this measurement, any imaging system can be used whose spatial resolution for imaging the robot achieves sufficient accuracy to detect and measure the spatial shape of the robot or a change in that shape. Preferably, the imaging system provides a 3D image of the robot.

Therefore, after the simultaneous execution of steps b), c), an information about the shape or the change of shape of the robot during the execution of its probing movement has been created. As said shape or the change of shape of the robot at least indirectly depends and/or is influenced by the properties of the material at the probing spot, in said information about the shape or the change of shape of the robot also information about the properties of the material at the probing spot is included.

This is used in the final step d) of the method according to the present invention. The data created by the imaging system while measuring the quasi-static and/or dynamical shape change of the robot measured in step c) is analyzed by a data analyzing system. Said data analyzing system can for instance include a customized model of the mechanics of the shape changeable member and/or of the whole robot. During the analysis, details and parts of the measured quasi-static and/or dynamical shape change of the robot can be extracted which cannot be explained and/or deduced by known properties of the shape changeable member and of the robot as a whole. Said remaining details and parts of the quasi-static and/or dynamical shape change of the robot can be used for deducing and finally determining the one or more properties of interest of the material.

In summary, after finishing step d) of the method according to the present invention, one or more properties of the material are determined based on the measurement carried out with the robot at the probing spot. As the robot is untethered, a fully remote measurement of said properties is possible, in particular also in cavities not easily accessible. Further, said measurement is not invasive and/or destructive.

There are many possible applications for the method according to the invention. For instance, but not limited to, the method can be implemented for investigating the inside of pipes of a sewage system and/or high-pressure lines of a power plant and/or containers for hazardous substances, such as toxic and/or flammable gases. Deposits, material fatigue or material change due to chemical reactions can be detected. As shape changeable members can be provided in various sizes, in particular down to 1 mm or less, also materials located in and/or accessible through very small cavities can be investigated. Another field of application of the method according to the present invention can be provided for biological and/or medical research and/or material examination, for instance the measurement of properties of materials present at the inner surface of the gastrointestinal tract of animals or human beings such as for instance gut tissue.

Especially in the latter example, the method according to the present invention allows avoiding usually used implanted electronic sensors, which also can be used for measuring physiological properties such as adhesion, pH, viscoelasticity, and biomarkers for disease diagnosis. However, these implanted electronic sensors are typically invasive requiring being deployed by surgery and frequently cause inflammation. All these disadvantages can be avoided by implementing the method according to the present invention.

Further, the method according to the present invention can comprise that for the determination in step d) also the second magnetic field and/or an expected response of the robot to the magnetic field is considered. The probing movement of the robot provided in step b) is a reaction of the shape changeable member on the actuation caused by the applied second magnetic field. The intrinsic response of the shape changeable member to said second magnetic field is known. In other words, it is known how the shape changeable member, and hence the robot as a whole, would react to an actuation by a specific second magnetic field without any external influence and/or boundary conditions such as interactions with the materials in the ambient environment of the robot. Therefore, by considering the second magnetic field and/or an expected response of the robot to the magnetic field, differences between a quasi-static and/or dynamical shape change of the robot expected based on the applied second magnetic field, and the actual quasi-static and/or dynamical shape change of the robot measured in step c) can be identified. Said differences can subsequently be used for deducing the one or more property of the material in step d) of the method according to the present invention.

Alternatively, or additionally, the method according to the present invention can be characterized in that for the one or more properties one or more members are selected from the group of members comprising adhesion, pH-value, elasticity, stiffness, friction, temperature, liquid wetting and/or viscoelasticity. This list is not closed, and also additional properties can be determined during the execution of the method according to the present invention. By measuring and/or investigating said properties, information about the condition of the probed material can be received. In other words, measuring said properties allows identifying existing or even upcoming problems when implementing and/or using the respective material. For instance, in pipes or hoses, properties as listed above change due to deposits on the wall of the pipes or hoses. Hence, based on properties determined by implementing the method according to the present invention decisions about needed maintenance or even replacement can be made. In biological system such as plants, animals or human bodies, the method according to the present invention allows determining these properties of a material in a not invasive way, for instance for research and science, but also as basic information for subsequently performed diagnostic procedures or treatments.

In addition, the method according to the present invention can comprise that for the imaging system one or more members are selected from the group of members comprising ultrasound, ay, X-ray fluoroscopy, photo-acoustic imaging, optical coherence tomography and/or optical imaging. This list it not closed, and also other suitable imaging systems can be used during execution of the method according to the present invention. All listed imaging systems are capable of providing images of the robot and hence can be used as basis for measuring the shape change of the robot in step c) of the method according to the present invention. In addition, put aside optical imaging, all listed imaging systems are capable of imaging the robot within a cavity from the outside of said cavity. By that the non-invasive characteristic of the method according to the present invention can be emphasized. However, also optical imaging can be optimized for minimizing invasive effects, for instance by miniaturization and/or the use of endoscopy technology.

In an additional, or alternative, embodiment of the method according to the present invention, the imaging system provides 1 or more, preferably 30 or more, more preferably 100 or more, images of the robot per second. In step c) of the method according to the present invention, a quasi-static and/or dynamical shape change of the robot is measured. However, said shape change can be a dynamical process, whereby not only the initial and final shape of the robot is of importance, but also the transition between said initial and final shape and/or the time structure of said transition. Hence, by using an imaging system capable of providing 1 or more, preferably 30 or more, more preferably 100 or more, images per second, time resolved information of the shape change can be measured and subsequently be considered when determining the one or more property of the material in step d) of the method according to the present invention.

Alternatively, or additionally, the method according to the present invention can be characterized in that the imaging system is used for locating the robot in step a). As mentioned above, in step a) the robot is guided to the intended probing spot of the material. As in many applications of the method according to the present invention said probing spot is on the inner surface of a cavity, monitoring the guiding of the robot by an imaging system is of advantage and/or even necessary. By using the imaging system already present for measuring the shape change of the robot in step c) of the method according to the present invention, providing an additional imaging system solely for monitoring the movement of the robot during step a) can be avoided.

Further, the method according to the present invention can be characterized in that in step a) the shape changeable member is actuated for successively creating different types of locomotion of the robot, in particular wherein the robot comprises a single shape changeable member formed as a sheet, preferably wherein the length of the sheet is at least three times the width of the sheet and the width of the sheet is at least 1 times, in particular at least 10 times, the height of the sheet, in particular a sheet of 6.5 mm × 2 mm × 0.15 mm. By providing different kinds of locomotion for the robot the range of the robot and hence the probing spots accessible by the robot can be enhanced. In particular, the first magnetic field can be altered for different responses and hence actuations of the shape changeable member, therefore resulting in different types of locomotion. For instance, the robot can move in different environments such as on more or less solid surfaces, on surfaces of liquids, and/or submerged in liquids. Preferably, this can be provided by a single shape changeable member formed as sheet. Sheet like in the sense of the present invention especially encloses all shapes with a length at least three times a width and height, respectively. Thereby the complexity and especially the size of the robot can be minimized, down to robots smaller than 1 cm.

Alternatively, and/or additionally, the method according to the present invention can comprise that in step a) for the type of locomotion of the robot one or more members are selected from the group of members comprising walking, climbing, crawling, rolling, sliding, stick-slipping, surface swimming, and/or submerged swimming. This list is not closed, and also additional types of locomotion can be provided by the robot during execution of the method according to the present invention. By selecting a suitable type of locomotion and by respectively providing the according first magnetic field for actuation of the shape changeable member, a type of locomotion suited best for the respective environment can be provided. Guiding the robot to the probing spot of the material can thereby be improved.

Also, in addition, or as alternative, the method according to the present invention can be implemented in such that the robot comprises two footpads arranged at opposite ends of the shape changeable member, and the robot further comprises an adhesive probing patch arranged along the shape changeable member between the two footpads. By providing footpads and probing patch, dedicated devices are provided as elements of the robot both for the movement of the robot in step a) and for the probing movement in step b) of the method according to the present invention. Both elements, the footpads and the probing patch, respectively, can be optimized for their respective tasks. An overall performance of the robot can thereby be enhanced. Further, by arranging the footpads at opposite ends of the shape changeable member, which additionally can preferably also be formed sheet-like, and by placing the probing patch between the footpads, a disruptive interaction between these elements can be avoided or at least minimized.

According to a first alternative embodiment, the method according to the present invention can comprise that the probing movement comprises a buckling motion of the robot, in particular for probing an adhesion and/or pH-value and/or elasticity and/or stiffness and/or friction and/or temperature and/or liquid wetting and/or viscoelasticity of the material, wherein as a first sub-step of the buckling motion the footpads are attached to the surface of the material at a distance to each other smaller than their distance along the shape changeable member for forming an arced shape of the robot, wherein for a subsequent second sub-step of the buckling motion the probing patch is brought into contact with the surface of the material while the footpads stay attached to the surface of the material, and wherein for a subsequent third sub-step of the buckling motion the probing patch is released from the contact with the surface of the material while the footpads stay attached to the surface of the material. In other words, in this embodiment of the method according to the present invention the material is investigated at the probing spot by controlled touching and releasing the probing patch at the surface of the material to be probed.

During the first sub-step of the buckling motion, a firm stand of the robot at the probing spot is established. The two footpads arranged at opposite ends of the shape changeable member are attached, preferably firmly attached or even fixed, at the material. Firmly attaching the footpads to the surface of the material to be probed can be provided by accordingly selecting the second magnetic field, inducing in the shape changeable member a force towards the surface. In particular, this is done in a manner that the shape changeable member, and hence the whole robot, is arched and hence towers arced shaped and u-shaped, respectively, over the probing spot.

As the probing patch is arranged between, preferably between in the middle, the two footpads, it faces the probing spot distanced to the surface of the material. Hence, in the next sub-step of the buckling motion, the second magnetic field is selected such that the part of the shape changeable member carrying the probing patch is lowered towards the surface, wherein finally the probing patch is brought into physical contact to the material. Preferably, the probing patch can be selected with respect to the one or more properties of the material to be probed. By that, the interaction between the probing patch and the surface of the material, in particular an adhesive force, can be made dependent on the properties to be investigated. As the two footpads stay attached to the surface of the material during the lowering of the probing patch, in summary this motion leads to a double-hump shape of the robot.

In the last sub-step of the buckling motion, the arced shape of the robot is restored. To achieve this, the probing patch is detached from the surface. As mentioned above, by accordingly selecting the material of the probing patch, the probing patch sticks to the surface depending on the one or more properties to be probed. Hence, especially releasing the probing patch from the surface and reveals information about said one or more property.

In summary, the buckling motion carried out in this embodiment of the method according to the present invention allows a dedicated and controlled contact and subsequent release of the probing patch on the material at the probing spot. By measuring the accompanying shape change of the shape changeable member, preferably additionally also measuring the second magnetic field, information about the adhesive force between the probing patch and the material can be obtained. As the probing patch is preferably suitably selected with respect to the one or more property to be investigated, these information about the adhesive force allows deducing the properties of interest.

The method according to the present invention can be enhanced further by that the probing patch covers 50% or less, in particular 10% or less, of a length and 10% or more, in particular 90% or more, of a width of the shape changeable member, respectively, wherein the probing patch is arranged at the shape changeable member centered between the two footpads, in particular wherein the probing patch has dimensions of 0.5 mm $\times$ 2 mm $\times$ 0.35 mm. As mentioned above, when the robot performs its bucking motion the probing patch is brought into contact with the surface of the material at the probing spot. By arranging the probing patch centered between the footpads, which are as already mentioned arranged at opposite ends of the shape changeable member, a symmetrical structure of the robot with same distances between the probing patch and each of the footpads can be provided, allowing a likewise symmetrical motion of the shape changeable member. The internal magnetic structure of the shape changeable member, which is responsible for its response to an external magnetic field, can thereby be simplified. Furthermore, as only a center region of the shape changeable member comes near to the surface of the material to be probed during the second sub-step of the buckling motion, limiting the size of the probing patch to this center region allows reducing the complexity, and also the cost, of the robot as a whole without limiting its ability to probe the material.

Additionally, or alternatively, the method according to the present invention can be enhanced by that the used probing patch comprises a pH-sensitive and/or protein-sensitive and/or mucus-sensitive and/or glucose-sensitive and/or temperature sensitive adhesion on the material to be probed. This list is not closed, and also probing patches with adhesions to additional properties of the material can be implemented during the execution of the method according to the present invention as mentioned above, the adhesion force between the probing patch and the surface of the material at the probing spot influences the interaction of the shape changeable member with the material. Consequently, the selection of the material of the probing patch cause measurable effects for the quasi-static and/or dynamical shape change of the robot. Therefore, by suitably selecting the probing patch to comprise a sensitive adhesion matched to the one or more properties to be investigated, the determination of said properties can be enhanced.

In a further enhanced embodiment of the method according to the present invention, in the second sub-step the second magnetic field is provided with a direction towards the surface of the material, and wherein in the third sub-step the magnetic field is provided with a direction away from the surface of the material. in particular wherein in the second sub-step and/or the third sub-step, the second magnetic field is provided perpendicular to the surface of the material. In the second sub-step of the buckling motion, the probing patch is brought towards the surface of the material of the probing spot. In contrast to that, in the third sub-step of the buckling motion, the probing patch is released from the contact with the surface, hence the probing patch moves away from the surface of the material at the probing spot. The shape of the shape changeable member, and hence its motion and the respective motion of the robot, can be adjusted by accordingly selecting the second magnetic field. A magnetic field towards and away from, respectively, the surface of the material at the probing spot can be provided easily and especially allows simplifying the internal magnetic structure of the shape changeable member. Said effects and advantages can be provided with a magnetic field generally directed towards and away from, respectively, the surface of the material at the probing spot, however, are most pronounced with a magnetic field perpendicular to said surface of the material.

The method can also, additionally, or alternatively, be enhanced by that in the second sub-step the second magnetic field is provided with a strength of 1 mT or more for 0.1 seconds or more. By applying a magnetic field with a strength of 1 mT or more for 0.1 seconds or more during the second sub-step of the buckling motion, a sufficient contact between the probing patch and the surface of the material at the probing spot can be provided. Preferably the respective strength of the second magnetic field is 500 mT or more, and/or the duration of the application is 5 seconds or more. Especially, the probing patch is pressed by the force induced by that specific magnetic field long enough that any physical and/or chemical effects influencing the above-mentioned adhesive force have settled. Hence, the shape change of the robot measured in step c) exclusively or at least predominantly depends on the adhesive force and therefore on the one or more property of the material to be probed.

Additionally, or alternatively, the method according to the present invention can also be enhanced by that in the third sub-step a strength of the magnetic field is gradually increased over time, in particular uniformly and/or step-wise, preferably wherein in the third sub-step a strength of the magnetic field gradually increased over 0.1 or more seconds, in particular 25 or more seconds, from 0 mT to 10 mT or more, in particular from 0 mT to 500 mT or more, in particular uniformly and/or in steps of 0.1 mT or more. In the third sub-step of the buckling motion, the second magnetic field is provided such that the contact between the probing patch and the surface of the material at the probing spot is released. However, the adhesive force between the probing patch and the surface of the material counteracts this release. By gradually increasing the strength of the second magnetic field, the necessary strength of the magnetic field for overcoming said adhesive force can be determined. This information can be used for enhancing the accuracy of the determination of the one or more properties of the material provided by the method according to the present invention.

According to a second alternative embodiment, the method according to the present invention can comprise that the probing movement comprises an undulating motion of the robot, in particular for probing a viscoelasticity of the material, wherein as a first sub-step of the undulating motion the footpads are attached to the surface of the material at a distance to each equal to their distance along the shape changeable member for bringing the robot in contact with the surface of the material along its entire length, wherein for a subsequent second sub-step of the undulating motion the shape changeable member is actuated for performing an oscillating motion while maintaining the contact with the surface of the material. In other words, in this embodiment of the method according to the present invention the material is investigated at the probing spot by a combined undulating motion of the robot and the material at the probing spot, wherein the shape change of the robot is directly influenced by the property, in particular the viscoelasticity, of the touched material.

For coupling the robot and the material at the probing spot, in the first sub-step of the undulating motion the robot is placed over its full length onto the surface of the material. For the respective movement of the robot, an accordingly selected suitable second magnetic field is provided. This can be provided by arranging the footpads at the probing spot at a distance corresponding to their distance measured along the shape changeable member, on which opposite ends the footpads are arranged. In other words, after the first sub-step of the undulating motion the robot lays flat on the surface of the material at the probing spot, touching the surface over its entire length. Thereby the probing patch is arranged, preferably pressed, to the surface of the material, effectively attaching the robot onto the material to be probed. An especially good physical connection between the robot and the material to be probed can thereby be provided.

Subsequently, in the second sub-step the provided second magnetic field is changed for inducing an oscillating motion in the shape changeable member, causing an undulating movement of the robot. Both, a standing wave and a propagating wave, respectively, along the shape changeable member are oscillating movements in the sense of the present invention. As the robot is and stays attached to the material over its entire length, the material at the probing spot is forced to follow this undulating movement of the robot. Thereby an overall movement is produced of the combined system consisting of robot and material, wherein said combined movement depends on properties of both the robot and the material. However, the properties of the robot are known, especially the expected response of the robot to the applied second magnetic field. By measuring the actual shape change of the robot and considering said known features of the robot, differences between the expected and the actual movement, and hence of the expected and actual shape change, of the robot, can be identified. Based on said differences, the one or more property to be probed of the material attached to the robot, especially its viscoelasticity, can be deduced and hence determined.

The method according to the present invention can be enhanced further by that the probing patch covers 90% or more of a length and 90% or more of a width of the shape changeable member, respectively, wherein the probing patch is arranged at the shape changeable member centered between the two footpads, in particular wherein the probing patch has dimensions of 6.5 mm × 2 mm × 0.05 mm. By providing a probing patch which extends over almost the complete, or even over the complete, surface of the shape changeable member, an especially good attachment of the shape changeable member and hence of the robot on the material at the probing spot can be provided. By arranging the probing patch centered between the footpads, which are as already mentioned arranged at opposite ends of the shape changeable member, a symmetrical structure of the robot with same distances between the probing patch and each of the footpads can be provided, allowing a likewise symmetrical motion of the shape changeable member. The internal magnetic structure of the shape changeable member, which is responsible for its response to an external magnetic field, can thereby be simplified.

According to another, additional or alternative, enhancement, the method according to the present invention can comprise that the used probing patch comprises an accordingly selected material which adheres to the material to be probed. During the oscillating motion of the shape changeable member in the second sub-step of the undulating motion of the robot, the shape changeable member, especially the probing spot, should stay firmly attached to the material at the probing spot. By accordingly selecting a suitable material for the probing patch, said firm attachment can be provided. Unintentional detachment of the probing patch from the surface of the material at the probing spot can thus be prevented or at least made significantly more difficult.

According to another enhanced embodiment, the method according to the present invention can comprise that in first sub-step, the probing patch and hence the robot is pressed against the surface of the material by an accordingly selected second magnetic field with a direction towards the surface of the material for 0.1 seconds or more, in particular for 30 seconds or more, preferably for 60 seconds or more, and/or with a force induced by the first magnetic field of 0.005 mN or more, preferably with a force of 0.1 mN or more. As mentioned above, for the deduction and determination of the one or more property of the material a firm attachment of the robot over its entire length on the surface of the material at the probing spot should be provided. By pressing the probing patch and hence the robot against said surface by an accordingly selected force, especially 0.005 mN or more, over a time period of 0.1 seconds or more, said firm attachment can be provided.

Additionally, or alternatively, the method according to the present invention can be characterized in that in the second sub-step the second magnetic field is provided with a constant strength and a changing direction, which is rotated perpendicular to an axis parallel to the width of the shape changeable member, in particular wherein the axis is arranged centered between the two footpads, in particular wherein the constant strength of the second magnetic field is between 1 mT and 500 mT, in particular between 10 mT and 30 mT, preferably is 20 mT. For providing the oscillating motion of the shape changeable member, which results in the undulating motion of the combined system of robot and material at the probing spot, the second magnetic field has to be time-dependently changed. A magnetic field with a constant strength and a simultaneously regularly changing direction has proven to be particularly suitable for this task, especially if the direction of the magnetic field is rotated perpendicular to an axis parallel to the width of the shape changeable member. The strength of the second magnetic field has to be strong enough to induce the undulating motion in both, the robot and the attached material, but simultaneously so small that losing the attachment of the robot on the surface is prohibited. A strength of the magnetic field between 1 mT and 500 mT, in particular between 10 mT and 30 mT, preferably of 20 mT, has been found especially suitable in this respect.

Again in addition, or as alternative, the method according to the present invention can comprise that a frequency of the rotation of the changing direction is selected between 0.005 Hz and 25 Hz, preferably is 0.1 Hz, in particular for probing a storage modulus of the material, in particular wherein for each selected frequency, the measurement of the dynamical shape in step d) is performed with said frequency kept constant for one or more, preferably 10 or more, more preferably 25 or more, complete rotations of the direction of the magnetic field. The viscoelasticity of a material can be expressed by the values of the storage modulus and the loss modulus of said material. For low frequencies of the rotation of the changing direction, the deformation induced by the robot into the material mainly depends only on the value of the storage modulus, and the effect of the loss modulus on said response of the material is negligible. The respective frequency value which can be considered as "low" in the sense of the present invention depends on the material to be probed, however a frequency value between 0.005 Hz and 25 Hz, preferably of 0.1 Hz, has been found most suitable. For the actual measurement, only this frequency is used and kept constant for one or more, preferably 10 or more, more preferably 25 or more, complete rotations of the direction of the magnetic field. The storage modulus can thereby be deduced with high accuracy.

Further, the method according to the present invention can be additionally, or alternatively, be enhanced by that the frequency of the rotation of the changing direction is stepwise increased with one steps or more, in particular 5 steps or more, selected within the interval from 0.003 Hz to 150 Hz, in particular for probing a loss modulus of the material, wherein the measurement of the dynamical shape in step d) is performed with for each of said frequency steps with the respective frequency kept constant for one or more, preferably 10 or more, more preferably 25 or more, complete rotations of the direction of the magnetic field. On the other hand, by selecting the frequency of the rotation of the changing direction in the interval from 0.003 Hz to 150 Hz in one or more, preferably several, steps, the response of the material to oscillating motions of the shape changeable member following these different frequencies of the second magnetic field can be measured. As the frequency increases, the influence of the loss modulus cannot be longer ignored. Hence, also the loss modulus can be extracted from the measured data, especially if in advance the storage modulus was already deduced for a low frequency measurement. Again, for the actual measurement, for each frequency step the respective frequency of the rotation of the direction of the magnetic field is kept constant for one or more, preferably 10 or more, more preferably 25 or more, complete rotations of the direction of the magnetic field. Also, the loss modulus can thereby be deduced with high accuracy.

According to another enhanced embodiment, the method according to the present invention can comprise that the storage modulus of the material and/or the loss modulus of the material is determined at two or more points, preferably determined continuously, along the length of the shape changeable member. In other words, a spatially resolved information about the storage modulus of the material and/or the loss modulus of the material, and hence of the viscoelasticity of the material, can be provided. In this way, for example, local hardening in an otherwise elastic material can be detected and precisely localized.

The method according to the present invention can further be characterized in that steps b) to d) are carried out repeatedly, in particular for 5 or more times. In other words, the measurement of the one or more properties of the material to be investigated are carried out at the same probing spot more than once, preferably for at least 5 times. By this, a statistic error when deucing a value for the respective property in question can be reduced. An overall accuracy of the measurement can be enhanced. Additionally, also step a) can be repeated between the respective repetitions of the combined execution of steps b) to d) for probing the one or more property of the material at another probing spot.

According to a second aspect of the invention the object is satisfied by a robot for probing one or more properties of a material at a probing spot at the surface of the material, wherein the robot is untethered and comprises a shape changeable member actuatable by temporally and/or spatially variable magnetic fields, wherein the robot comprises two footpads arranged at opposite ends of the shape changeable member, and the robot further comprises an adhesive probing patch arranged along the shape changeable member between the two footpads. The robot according to second aspect of the present invention is characterized in that the robot is constructed to be used during execution of the method according to one of the preceding claims. Hence, the robot can comprise the same features and advantages described above with respect to the method according to the first aspect of the present invention.

In addition, the robot according to the present invention can be characterized in that the robot comprises only one shape changeable member formed as a sheet, wherein the length of the sheet is at least three times the width of the sheet and the width of the sheet is at least 1 times, in particular at least 10 times, the height of the sheet, preferably wherein the shape changeable member comprises a thickness-to-length ratio of 0.023, in particular wherein the shape changeable member is a sheet of 6.5 mm × 2 mm × 0.15 mm. By only using a single sheet-like changeable member, the robot according to the present invention can be constructed especially simple, and additionally also small, especially smaller than 1 cm. By using a shape changeable member comprising a thickness-to-length ratio of 0.023, in particular by using a shape changeable member which is a sheet of 6.5 mm × 2 mm × 0.15 mm, it has been shown that a robot can be provided capable of different types of movements and locomotion, especially waking, climbing and also performing a buckling motion.

Alternatively, or additionally, the robot according to the present invention can comprise that the footpads comprise a hollow-cylinder-shaped body, wherein on the body microspikes coated with hydrogel and chitosan-based bio-adhesives are arranged. By providing footpads with said coated microspikes, attaching and detaching to the surface of the material to be investigated can be controlled by external magnetic fields in an excellent way. For instance, the microspikes can be arranged in a 9 × 7 array of conical spikes of 200 μm in height, 100 μm in diameter and 200 μm in spacing. Further, said arrays of microspikes were layered by the hydrogel and chitosan-based bio-adhesives.

Further, the robot according to the present invention can also be enhanced by that the adhesive probing patch comprises a three-layer structure, a layer of elastomer, preferably with a thickness of 0.1 mm, facing the shape changeable member, on top of that a layer of coated hydrogel, preferably with a thickness of 0.05 mm, and on top of that a layer of pH-sensitive and/or protein-sensitive and/or mucus-sensitive and/or glucose-sensitive and/or temperature sensitive bio-adhesive, preferably with a thickness of 0.2 mm, wherein in particular the probing patch has dimensions of 0.5 mm × 2 mm × 0.35 mm. Said structure of the probing patch has been found most suitable for an implementation of the method performing a buckling motion as probing movement. The respective probing patch adheres to the surface at the probing spot depending on the used bio-adhesive, automatically gaining information about the property of the material to be probed.

Alternatively, the robot according to the present invention can comprise that the adhesive probing patch comprises a two-layer structure comprising a hydrogel layer and a layer of chitosan-based bio-adhesives, preferably with a total thickness of 0.05 mm, wherein in particular the probing patch has dimensions of 6.5 mm × 2 mm × 0.05 mm. Chitosan-based bio-adhesives, which can also be used for the footpads of the robot, provide an especially general good adhesion to the material at the probing spot. This is especially useful for an implementation of the method based on an undulating motion of the whole robot which is and should stay firmly attached to the material at the probing spot, especially over the whole length of the robot.

As described above, for both implementation of the method according to the present invention, one based on a buckling motion of the robot, the other based on an undulating motion of the robot, a specially designed probing patch can be provided. However, according to another embodiment of the robot according to the present invention it is also possible that the robot comprises both the probing patch with a three-layer structure suitable for the buckling motion and the probing patch with a two-layer structure suitable for the undulating motion, wherein the different probing patches are arranged on opposite sides of the shape changeable member. A robot well suited for both implementations of the method according to the first aspect of the present invention can thereby be provided.

In a third aspect of the invention, the object is satisfied by a system for probing one or more properties of a material at a probing spot at the surface of the material, comprising a magnetic actuation system, an imaging system, a data analyzing system, and a robot according to the second aspect of the present invention. The system according to the third aspect of the present invention is characterized in that the system is constructed for carrying out the method according to the first aspect of the present invention. Hence, all features and advantages described above with respect to the method according to the first aspect of the present invention, and to robot according to the second aspect of the present invention, respectively, can also be provided by the system according to the third aspect of the present invention.

The invention will be explained in detail in the following by means of embodiments and with reference to the drawings. In particular, in the figures are shown:

Fig. 1    Probing of material properties by an untethered miniature soft robot,

Fig. 2    Mechanism of probing material properties by an untethered miniature soft robot,

Fig. 3    Probing adhesion and pH using an untethered miniature soft robot comprising a pH-sensitive adhesive probing patch,

Fig. 4    Quantification of the performance of probing robot-material adhesion and therein the pH,

Fig. 5    Probing material viscoelasticity using an untethered soft robot comprising a bio-adhesive probing patch,

Fig. 6    Quantification of the performance of probing viscoelasticity,

Fig. 7    Demonstration of probing properties of a material by measuring soft tissue properties in an ex vivo intestinal disease model using X-ray medical imaging,

Fig. 8    Customized experimental setup for the adhesion measurement,

Fig. 9    Effect of the thickness-to-length ratio of the robot and the shape changeable member, respectively, design on the robot locomotion and probing performance,

Fig. 10    The estimated force at the adhesive probing patch and the corresponding external magnetic field of the robot adhesion probing over 5 repetitions,

Fig. 11    The estimated robot-tissue adhesion on different tissue surfaces of various pH values,

Fig. 12    Robot deformation under dynamic actuation for probing viscoelasticity,

Fig. 13    Effect of the substrate material thickness on the viscoelasticity probing,

Fig. 14    Correction of the misalignment between the coordinate systems of the imaging plane and the robot,

Fig. 15    Schematics of the robot probing viscoelasticity of the non-homogeneous materials,

Fig. 16    Robot deformation in the presence of the stiff cylinder of various sizes at different locations under dynamic actuation for viscoelasticity probing,

Fig. 17    Material deformation distribution in the presence of the stiff cylinder of various sizes at different locations during viscoelasticity probing,

Fig. 18    Estimation of the cylinder location in the x axis and quantification of its influence on the robot deformation with the wavelet analysis during viscoelasticity probing,

Fig. 19    Probing tissue viscoelastic properties by robot dynamic shape under ultrasound imaging,

Fig. 20    The basal pH in the stomach of the TNK1-expression mouse and the mouse from the healthy control group,

Fig. 21    The effect of the detection noise on the adhesion estimation,

Fig. 22    The estimated maximum reaction torque at the adhesive probing patch and the substrate material interface during adhesion probing,

Fig. 23    Determination of the correlation coefficient c between the robot displacement and the strain field of the substrate material for viscoelasticity probing,

Fig. 24    The electromagnetic actuation system for controlling the soft robot,

Fig. 25    Experimental setup for probing viscoelasticity of bulk materials using wireless soft robots, and

Fig. 26    The experimental setups for probing adhesion and viscoelasticity inside an X-ray cabinet.

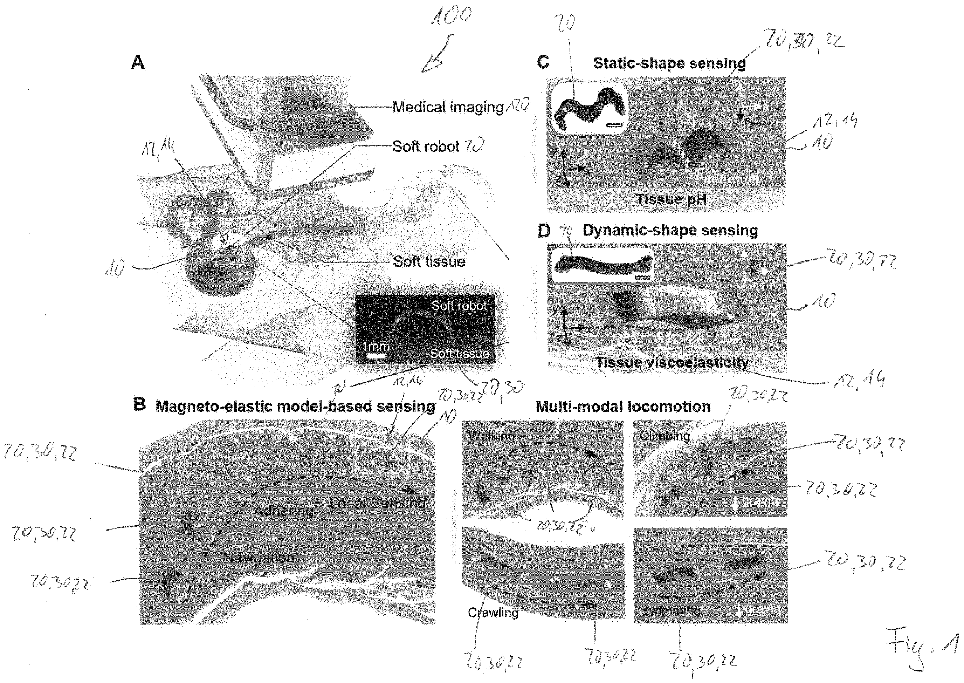

In the figures 1 to 26, possible embodiments of systems 100, robots 20, and methods, respectively, according to the present invention and some additional supplementary information are shown. In the depicted embodiments, probing soft tissue of animal or human bodies is the main focus. However, the present invention and its aspects, the method according to the first aspect of the present invention, the robot 20 according to the second aspect of the present invention, and the system 100 according to the third aspect of the present invention, respectively, are not limited to this field of action but can also be used for probing all kinds of materials 10, for instance pipes, hoses, and/or fabrics. All general concepts described in the following with respect to probing soft tissue can also be implemented for probing other materials 10, especially other non-biological materials 10.

The method according to the first aspect of the present invention can be carried out by a system 100 according to the third aspect of the present invention. Said system 100 comprises four key components for probing one or more properties of a material 10 at a certain probing spot 14.

First of all, the system 100 comprises a robot 20 according to the second aspect of the present invention. Said robot 20 is untethered and comprises a shape changeable member 30 actuatable by temporally and/or spatially variable magnetic fields. Further, the robot 20 comprises two footpads 22 arranged on opposite ends of the shape changeable member 30. In particular, the footpads 22 can comprise a hollow-cylinder-shaped body 24, wherein on the body 24 microspikes coated with hydrogel 62 and chitosan-based bio-adhesives 64 are arranged. Preferably, the robot 20 comprises only one shape changeable member 30 formed as a sheet 34, wherein the length 36 of the sheet 34 is at least three times the width 38 of the sheet 34 and the width 38 of the sheet 34 is at least 1 time, in particular at least 10 times, the height 40 of the sheet 34. In the figures, the shape changeable member 30 comprises a preferred thickness-to-length 36 ratio (TLR) of 0.023. For providing this particular TLR, the shape changeable member 30 can be provided as a sheet 34 of 6.5 mm $\times$ 2 mm $\times$ 0.15 mm.

As described in detail below, for different probing procedures, different adhesive probing patches 50 can be attached to the robot 20, in particular on the shape changeable member 30, preferably in the center between the two footpads 22. For instance, the adhesive probing patch 50 can comprise a three-layer structure, a layer of elastomer 60, preferably with a thickness of 0.1 mm, facing the shape changeable member 30, on top of that a layer of coated hydrogel 62, preferably with a thickness of 0.05 mm, and on top of that a layer of pH-sensitive and/or protein-sensitive and/or mucus-sensitive and/or glucose-sensitive and/or temperature sensitive bio-adhesive 64, preferably with a thickness of 0.2 mm. Such a three-layered probing patch 50 preferably has dimensions of 0.5 mm $\times$ 2 mm $\times$ 0.35 mm. As an alternative embodiment, the adhesive probing patch 50 can comprises a two-layer structure comprising a hydrogel 62 layer and a layer of chitosan-based bio-adhesives 64. A total thickness of such a two-layered probing patch 50 is 0.05 mm, preferred dimensions are 6.5 mm $\times$ 2 mm $\times$ 0.05 mm.

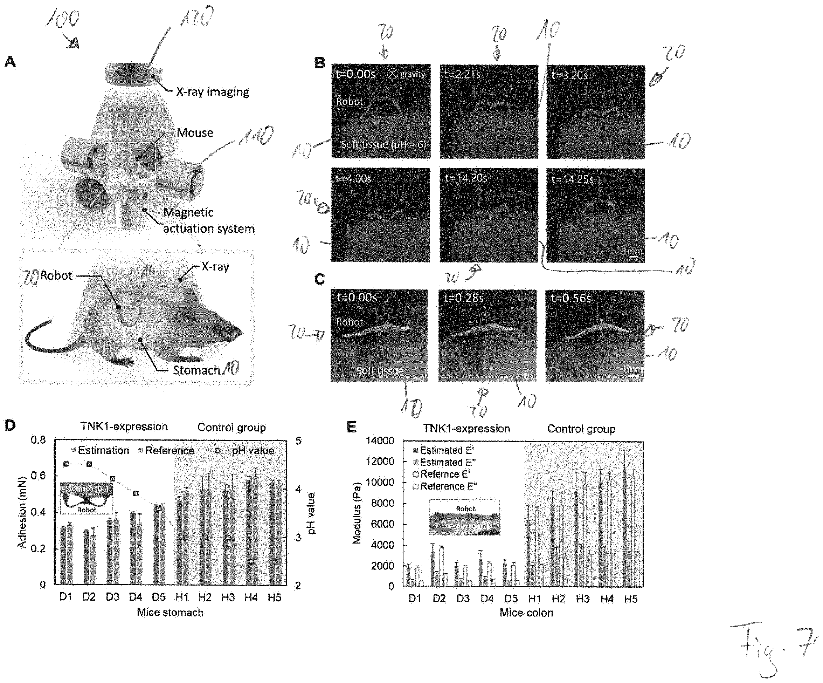

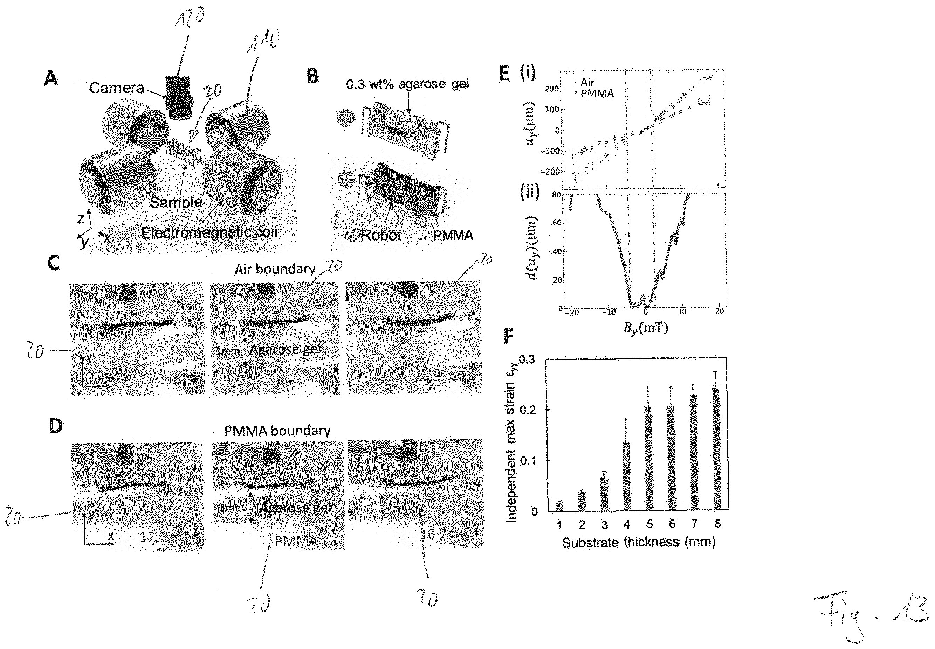

As second component, for generating said magnetic fields, the system 100 comprises a magnetic actuation system 110. The magnetic actuation system 110 can for instance comprise 4 (see Fig. 13) or 6 (see Fig. 7) magnetic coils aligned along 2 or 3 axes perpendicular to each other. In particular, said magnetic actuation system 110 can provide temporally and/or spatially variable magnetic fields preferably for both, travelling movements of the robot 20, in particular towards the probing spot 14, and probing movements of the robot 20 at the probing spot 14.

Further, an imaging system 120 as third component of the system 100 allows precise tracking of the robot 20, especially when the robot 20 is used in cavities which are not directly accessible. Said imaging system 120 can be selected from the group of members comprising ultrasound, X-ray, X-ray fluoroscopy, photo-acoustic imaging, optical coherence tomography and/or optical imaging.

Finally, the system 100 according to the present invention comprises a data analyzing system 130 for extracting and deducing the desired information about the one or more properties of the material 10. For that, the image information provided by the imaging system 120 about a quasi-static and/or dynamic shape change of the robot 20 and/or the shape changeable member 30 is analyzed. A specific model of the dynamics of the robot 20 can preferably be implemented in the data analyzing system 130. Additionally, the characteristics of the magnetic fields provided by the magnetic actuation system 110 can also be considered during the analysis.

In short, the method according to the present invention comprises four steps:

Step a): The robot 20 is guided to the probing spot 14 on the surface 12 of the material 10 to be probed by providing a first magnetic field by the magnetic actuation system 110 with an accordingly selected time and spatial characteristic for actuating the robot 20 in one or more types of locomotion;

Step b): A second magnetic field is provided by the magnetic actuation system 110 with an accordingly selected time and spatial characteristic for actuating the robot 20 to carry out a probing movement;

Step c): A quasi-static or dynamical shape change of the robot 20 is measured by the imaging system 120 simultaneously to step b); and

Step d): The one or more property of the material 10 is determined by the data analyzing system 130 by evaluating the measurements of the imaging system 120, preferably by considering the characteristics of the second magnetic field.

The untethered and hence wirelessly actuated soft robot 20, an imaging system 120 provided as medical imaging module, such as ultrasound imaging or X-ray fluoroscopy machines, for tracking the robot 20 shapes, and the material 10 to be probed, in the depicted case a targeted soft tissue is shown in Fig. 1A.

The robot 20 soft body shape is precisely controlled by external magnetic fields generated by a customized magnetic actuation system 110. As shown in Fig. 1B, the robot 20 has multimodal locomotion modes, including walking and climbing on soft tissue surfaces 12, crawling in narrow crevices, and swimming on the surface 12 of or inside liquids, to navigate through complex terrains and attach to soft tissue surfaces 12 by a bio-adhesive 64 probing patch 50 bonded to the robot 20, in particular on the shape changeable member 30. Other possible types of locomotion include rolling, sliding, and/or stick-slipping.

For guiding the robot 20 during step a), the imaging system 120 can be used.

Two types of robot 20-tissue-interaction are reported, including quasi-static and dynamic interactions. In the quasi-static interaction, the robot 20 interacts with the soft tissue in a quasi-static manner, where the damping force is negligible, as showcased with a buckling-based motion in Fig. 1C.

In the dynamic interaction, the robot 20 constantly experiences a time-varying magnetic field, resulting in a dynamic deformation of the material 10 surface 12, as depicted the tissue surface 12, which depends on both the magnitude and frequency of the external magnetic fields. Figure 1D shows that the robot 20 can be attached over its whole length 36 to the material 10 (soft tissue) surface 12 and perform an undulating motion.

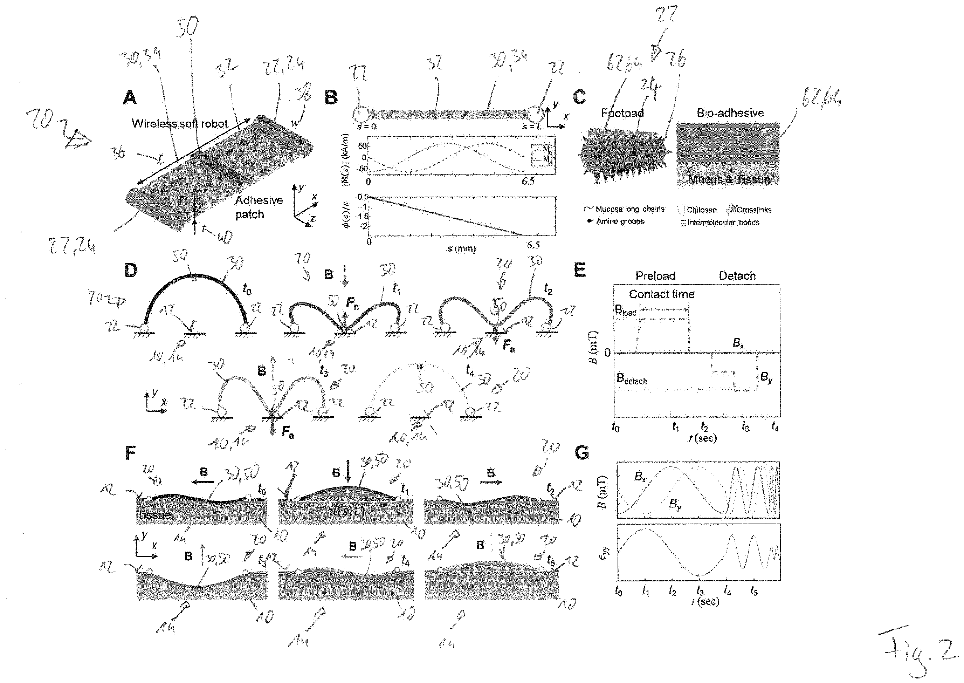

For probing properties of material 10 such as soft tissue, an untethered, wireless soft robot 20 is provided by bonding bio-adhesive 64 probing patches 50 on the shape changeable member 30 of the robot 20. The applied control strategies allow the robot 20 to interact with soft tissues for in-situ probing. As shown in Fig. 2A, the robot 20 has a magnetoelastic soft main element formed by a single sheet-like shape changeable member 30, two ring-shaped footpads 22 on its opposite ends, and an adhesive probing patch 50 bonded to the shape changeable member 30.

The robot 20, and the shape changeable member 30, respectively, (length 36: L, width 38: w, thickness: t) has magnetization magnitude and phase profiles $M(s)$ and $\phi(s)$ ($s \in [0, L]$) as shown in Fig. 2B, which allow it to realize desired multimodal locomotion, including climbing, crawling, and swimming (23).

The footpads 22 have microspikes coated with hydrogel 62 and tough bio-adhesives 64 made of chitosan, as shown in Fig. 2C, so that it can attach to and detach from tissue surfaces 12 controlled by external magnetic fields provided by the magnetic actuation system 110.

Two fundamental mechanisms have been proposed for probing material 10 properties, in particular tissue physiological properties, in situ. First, the robot 20 can use buckling-based static shapes to sense robot 20-material 10 adhesion (Fig. 2D). Such adhesion measurement can also be used for probing various mechanical properties of the material 10 or tissue, such as pH and temperature, by using stimuli-responsive adhesives.

A remote magnetic field (Fig. 2E), provided by the magnetic actuation system 110, is controlled to induce the buckling motion, which will be discussed in detail in the following. The fundamental principle is that when the two footpads 22 of the robot 20 attach to a soft tissue surface 12, a magnetic field B perpendicular to the tissue surface 12 in the negative y direction will induce the deformation of the robot 20 towards the tissue surface 12. The adhesive probing patch 50 will be loaded to the tissue surface 12 due to a buckling effect.

While after removing the magnetic field, the robot 20 will maintain the buckled shape due to the patch-tissue adhesion. Gradually increasing the magnetic field in the positive y direction will result in a distributed magnetic torque to detach the adhesive probing patch 50 from the tissue surface 12 once the B field reaches a threshold value. Moreover, the patch-tissue adhesion can be estimated using the robot 20 and the shape changeable member 30, respectively, shape and the input magnetic field based on a magnetoelastic model using the Euler-Bernoulli Beam theory (see below).

The static shape of the robot 20 and the shape changeable member 30, respectively, is described by $\theta(s)$ and $\frac{\partial \theta}{\partial s}$, which are the slope angle and curvature of the robot 20 and the shape changeable member 30, respectively, at a specific location $s \in [0, L]$, respectively. The shape data can be obtained by optical imaging in vitro and medical imaging in vivo, while the input magnetic field can be obtained from the calibrated magnetic actuation system 110.

In addition, our robot 20 can also adhere to and dynamically interact with materials 10 such as soft tissues to sense their viscoelastic properties under a periodic magnetic field waveform (Fig. 2F). For example, Fig. 2G shows rotating magnetic fields with a constant magnitude at various frequencies. The tissue viscoelastic properties can be estimated from the tissue normal strain $\epsilon_{yy}^{t}$ and the tissue normal stress $\sigma_{yy}^{t}$ at the robot 20-tissue interface. The soft tissue is approximated with a first-order dynamic system 100, where the relationship between the normal stress $\sigma_{yy}^{t}$ and the normal strain $\epsilon_{yy}^{t}$ is given by $\sigma_{yy}^{t} = k_e E' \epsilon_{yy}^{t} + k_e k_\tau E'' \frac{d\epsilon_{yy}^{t}}{dt}$, where $E'$ and $E''$ are the storage and loss moduli of the tissue, respectively, while $k_e$ and $k_\tau$ are the coefficients, and $\epsilon_{yy}^{t}$ is a function of the tissue normal displacement $u_y^{t}$.

At the robot 20-tissue interface, we have $\sigma_{yy}^{t} = - \sigma_{yy}^{r}$ and $u_y^{t} = u_y^{r}$, where $\sigma_{yy}^{r}$ and uy are the robot 20 normal stress and displacement, respectively. We can further use the dynamic shape and/or shape change of the robot 20 to approximate the tissue strain and stress using a magneto-elastic model. Given a specific robot 20, the normal stress of the soft tissue is assumed to be proportional to the external magnetic field with a scaling factor $k_y$ assuming small deflections. When subjected to a rotating magnetic field with a frequency of f = $\omega/2\pi$, $\sigma_{yy}^{r}$ can be estimated from the magnetic field given by $\sigma_{yy}^{r} = k_y(M(s), K_r)B(\omega t)$, where $k_y$ is a function of the bulk modulus $K_r$ and the magnetization profile M(s) of the robot 20, which are independent of the external magnetic field and the soft tissue properties. Therefore, if a frequency sweeping for the function $\epsilon_{yy}^{t}(\omega t)/B(\omega t)$ by varying $\omega$ is performed, $E'$ and $E''$ of the soft tissue using the low and high frequency parts of the frequency response can be estimated, as described in detail below.

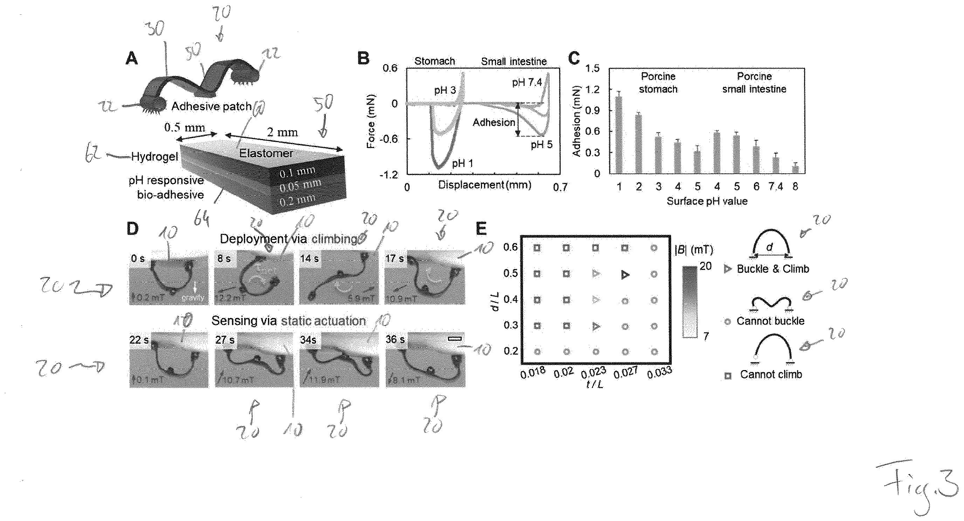

In Fig. 3, we present the robot 20 design, characterization, probing mechanism, and validation results for probing robot 20-tissue adhesion and therein the tissue pH values at a specific location. A 2 mm × 0.5 mm × 0.35 mm adhesive probing patch 50 coated with pH-sensitive bio-adhesives 64 is bonded at the middle of the robot 20 and the shape changeable member 30, respectively, (Fig. 3A). The patch is composed of three layers: a layer of elastomer 60 (thickness: 0.1 mm), a thin layer of coated hydrogel 62 (thickness: 0.05 mm), and a layer of pH-sensitive bio-adhesive 64 (thickness: 0.2 mm). The hydrogel 62 layer serves as a dissipative matrix material 10 for tough adhesives as well as a bonding material 10 between the elastomer 60 patch and bio-adhesive 64. The pH-sensitive bio-adhesive 64 layer combines both the chitosan-based bio-adhesive 64 for tough adhesion to mucus-covered tissues and the catechol-boronate-based hydrogel 62 adhesive for pH-responsive adhesion.

The pH-sensitive adhesion to tissue surfaces 12 of the adhesive probing patch 50 can be quantified, see Figs. 3B, C. Fig. 3B compares the adhesion of the adhesive probing patch 50 on ex-vivo porcine stomach and small intestine tissue surfaces 12 with different pH values. Standard phosphate-buffered saline (PBS) buffer solutions with different pH values are added to the same tissue surfaces 12 to achieve the desired pH values, validated by a pH indicator. With a preload of 0.5 mN in a customized adhesion measurement setup (Fig. 7), we perform a systematic 100 measurement of the patch-tissue adhesion. The adhesive probing patch 50 shows adhesion of 1.08±0.07 mN on an acidic porcine stomach surface 12 with pH = 1, but only 0.54±0.06 mN on the same porcine stomach surface 12 with pH = 3. Furthermore, the adhesive probing patch 50 shows adhesion of 0.56±0.04 mN on a porcine small intestine surface 12 with pH = 5, while only 0.23±0.09 mN on the same porcine small intestine surface 12 with pH = 7.4.

In Fig. 3C, it is depicted that the patch-tissue adhesion is almost linearly dependent on the pH of the tissue surface 12, so that the patch-tissue adhesion can be used to sense pH assuming similar surface 12 conditions. First, the pH-responsive properties of the adhesive probing patch 50 can be designed to sense a wide range of pH values. For example, the adhesion decreases from 1.08±0.07 mN to 0.31±0.12 mN on the porcine stomach tissue surfaces 12 when the pH increases from 1 to 5. This pH range can cover most of the possible cases in the stomach. For example, the average pH is about 5.4 ±2.1 in patients with gastric cancer, 3.0 ±2.2 for gastritis, 2.4 ±1.9 for gastric ulcers, 1.3 ±0.6 for duodenal ulcers, and 1.7 ±0.2 for normal subject. Meanwhile, the adhesion decreases from 0.58±0.03 mN to 0.11±0.05 mN on porcine small intestine tissue surfaces 12 when a pH increases from 4 to 8. In addition, our pH-responsive adhesive probing patch 50 shows a comparable sensitivity of 1 pH unit like the commercial pH-indicator strips pH = 2.0 - 9.0 with a probing range from 1 to 8.

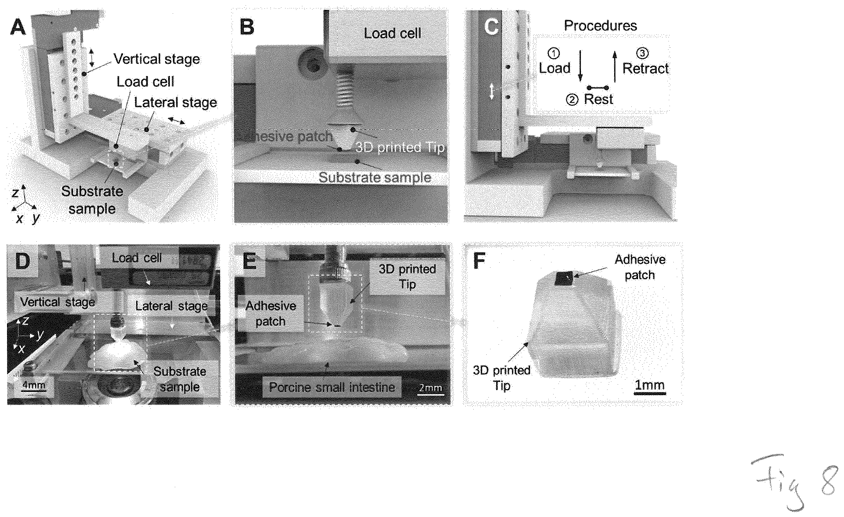

Figure 3D shows the design of the robot 20 for achieving both desired climbing locomotion and the adhesion probing function. To achieve both climbing locomotion and deployment for probing, the robot 20 and the shape changeable member 30, respectively, was optimized with a key design parameter - the thickness-to-length 36 ratio (TLR), which compromises the climbing locomotion and the buckling-based attachment to tissues. With a relatively small TLR, the robot 20 can be deployed with a buckling motion easily due to a relatively small bending stiffness, but the robot 20 has degraded climbing locomotion due to insufficient net magnetic torque (Fig. 8).

A systematic 100 parameter sweeping of the TLR from 0.018 to 0.033 (body thickness: 0.15 mm) was performed to optimize the TLR for both the robot 20 climbing locomotion and the buckling-based attachment to tissues. We find that a TLR of 0.023 (body length 36: 6.5 mm) allows a sufficient climbing ability allowing a wide range of reachability quantified by the footpad 22-distance-to-length 36 ratio (0.3 to 0.5) while using a relatively small actuation magnetic field (< 20 mT). Meanwhile, the same robot 20 also allows easy deployment of the robot 20 and the shape changeable member 30, respectively, as showcased in Fig. 3E.

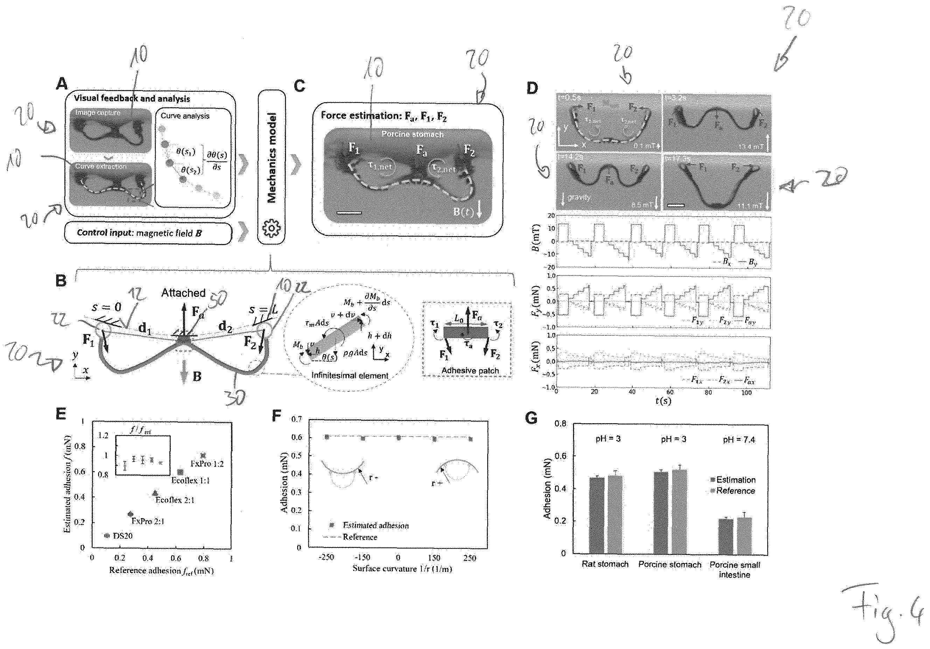

Figure 4A shows the schematics of estimating the robot 20-tissue adhesion using a magnetoelastic model. Given the robot 20 static shapes parameterized by $\theta(s)$, $\dfrac{\partial \theta(s)}{\partial s}$ and the external magnetic field B, the patch-tissue adhesion can be estimated.

Figure 4B illustrates the free-body diagram of the robot 20 under a "pinned-fixed-pinned" boundary condition, where the adhesion $F_a$ between the adhesive probing patch 50 and the tissue surface 12 is balanced by the reaction forces $F_1$ and $F_2$ in addition to the robot 20 weight.

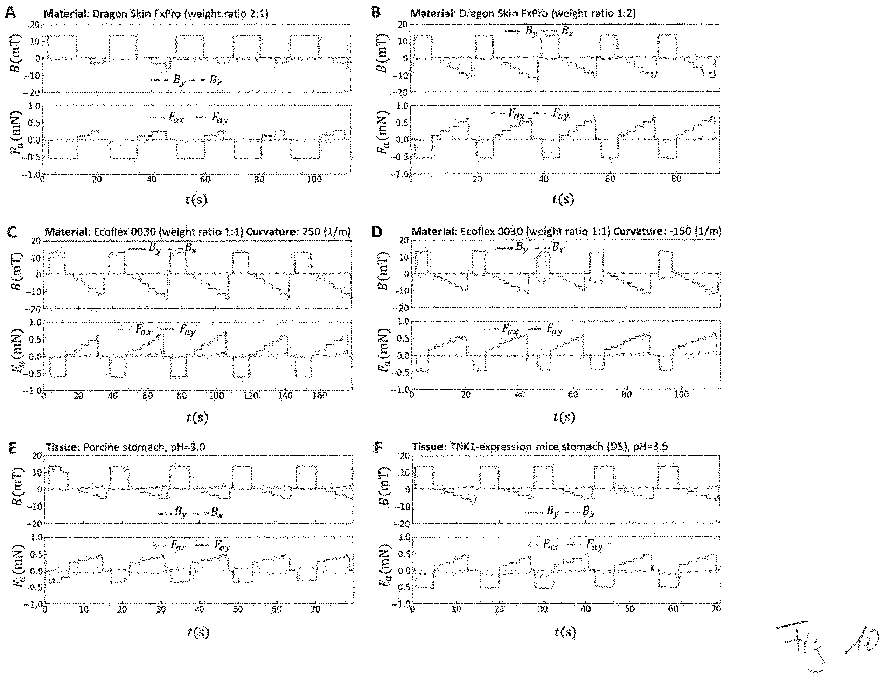

As shown in Fig. 4C, $F_1$, $F_2$ and $F_a$ can be estimated by solving the moment and force balance equations based on the Euler-Bernoulli beam theory (see below). As an example, Figure 4D illustrates the probing process of the robot 20-material 10 adhesion $F_a$ on a substrate surface 12 made of Ecoflex-0030 silicone rubber (1:1 weight ratio). In this process, the robot 20 is first loaded for attachment with an external magnetic field of 13.4 mT for 5 sec in the +y axis. Then, an external magnetic field in the -y axis is applied from 0 mT with a magnitude interval of 2.7 mT and a time interval of 2.5 sec until the robot 20 is detached from the surface 12. Note that this procedure yields consistent adhesion estimation results for over 5 repetitions with an average relative standard deviation of 4.4%, as shown in Fig. 10.

Further, the estimated adhesion using the soft robot 20 on various synthetic substrate surfaces 12 are compared with the adhesion measured by a high-precision force sensor in a customized adhesion measurement setup (Fig. 7). Figure 4E compares the estimated adhesion and the reference adhesion for synthetic surfaces 12 with various adhesion properties. For the adhesion range from 0.13±0.02 mN to 0.82±0.02 mN, the estimated results from the probing method according to the present invention match the reference adhesion with relative errors all less than 12%, as shown in Fig. 4E.

The probing method according to the present invention is also applicable to a substrate surface 12 with relatively large curvatures. Probing experiments were performed on convex, flat, and concave surfaces 12 with a curvature of -250, -150, 0, 150, and 250 m$^{-1}$ all covered by the 2 mm-thick Ecoflex-0030 silicone rubber sheets (1:1 weight ratio). The relative errors between the estimated adhesion on curved surfaces 12 and the measured force are less than 2.6% (Fig. 4F). In summary, the probing method according to the present invention can estimate the robot 20-substrate adhesion with a relative error less than 20% for a wide range of adhesion from 0.13±0.02 mN to 0.82±0.02 mN and various curvatures from -250 to 250 m$^{-1}$.

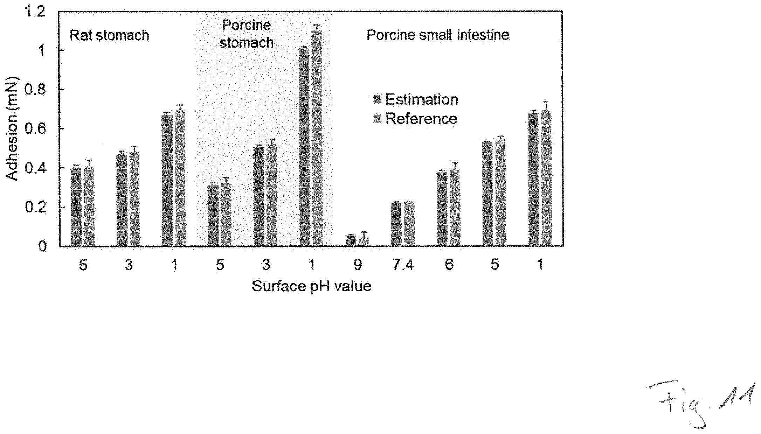

To further quantify the performance of probing robot 20-tissue adhesion and therein the pH values, the robot 20 was used to probe adhesion on various tissue surfaces 12 with different pH values. Figure 4G compares the estimated adhesion by the probing method according to the present invention and the reference adhesion measured by a high-precision force sensor on materials 10, in particular tissues, including rat stomach, porcine stomach, and porcine small intestine (see Fig. 11 for more detailed comparisons for the same tissues covered by PBS solutions of various pH values). The relative errors in adhesion between the estimation and the measured values for all three different tissues are less than 10%. Therefore, the probing method according to the present invention could potentially enable minimally invasive probing of the tissue adhesion and pH at a hard-to-reach probing spot 14, for instance inside the human body with the aid of medical imaging systems 120.

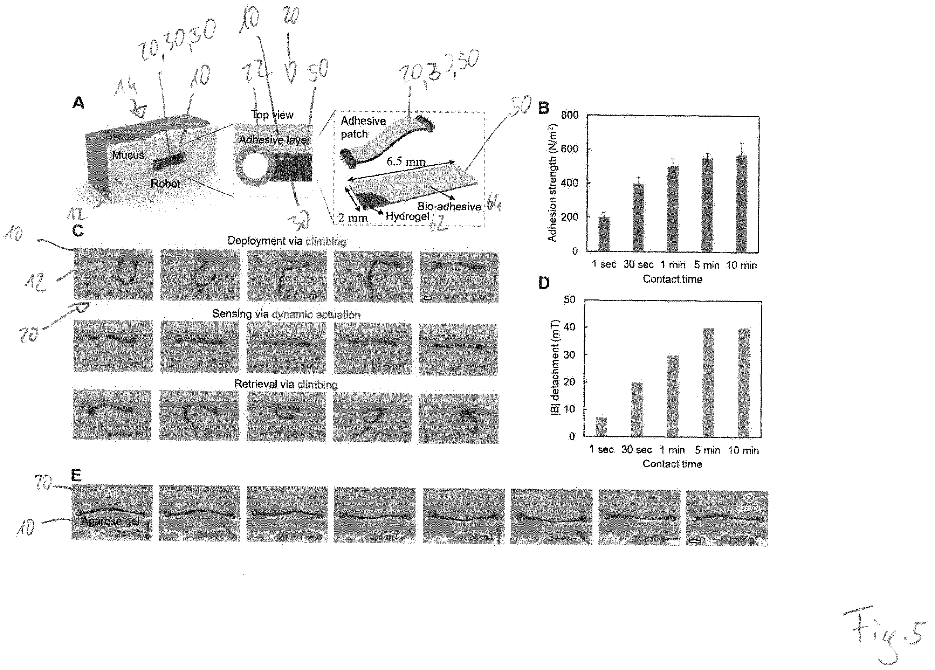

In Fig. 5, the design of the soft robot 20 to sense tissue viscoelastic properties with dynamic robot 20 motion is presented. To maintain the robot 20-tissue attachment during the dynamic interactions, a 6.5 mm × 2 mm adhesive probing patch 50 is bonded to the shape changeable member 30, and hence to the robot 20 (Fig. 5A). The adhesive probing patch 50 is fabricated by coating the robot 20 and the shape changeable member 30, respectively, with hydrogel 62 and bio-adhesive 64 sequentially with a total thickness of about 0.05 mm. The adhesive layer bonds the robot 20 and tissue surfaces 12 together while having a minimal effect on the robot 20-tissue dynamic interactions.

Further, the adhesion strength between the robot 20 and tissue surfaces 12 can be adjusted by the contact time, see Fig. 5B. The adhesion between the bio-adhesive 64 probing patch 50 and the porcine small intestine tissue used as material 10 to be probed is a function of the contact time, characterized by a customized setup with a preload of 0.1 mN (Fig. 7). The bonding process involves the intermolecular interaction and crosslinking between the bio-adhesive 64 and the mucus, which is time-dependent. When the bonding is sufficiently formed (after 1 min), the adhesion per unit area is around $509 \pm 47$ N/m$^2$ and large enough to maintain the robot 20-tissue attachment. Additionally, the adhesion per unit area is still within $574 \pm 83$ N/m$^2$ as the contact time reaches 10 min, enabling the on-demand detachment under magnetic actuation.

Figure 5C shows the robot 20 attachment, probing, and detachment behaviors for probing viscoelasticity of a material 10, such as for example tissue. First, the robot 20 is deployed and adheres its bio-adhesive 64 side to the targeted tissue area with the whole-body torque generated by the nonzero net magnetic torque $\tau_{net}$. Then, the robot 20, in particular its shape changeable member 30, is actuated to sense tissue viscoelasticity under a rotating magnetic field (magnitude: 7.5 mT). When the probing process is completed, the robot 20 and the shape changeable member 30, respectively, is detached from the tissue surface 12 under a magnetic field with a relatively large magnitude (~26 mT).

Figure 5D shows the minimum magnetic field needed for the detachment as a function of the contact time when the robot 20 is attached to the porcine small intestine tissue. A more detailed illustration of the robot 20 dynamic motion under a rotating magnetic field is presented in Fig. 5E, where the robot 20 undulates when attaching to an agarose gel (0.3 wt%) back layer under a rotating magnetic field (24 mT, 0.1 Hz).

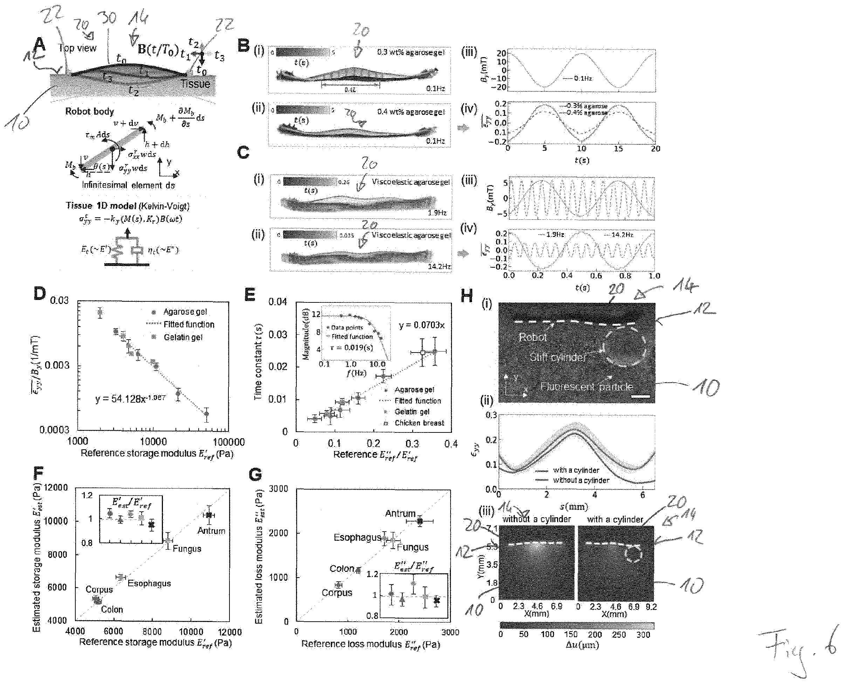

In Fig. 6, we further show the method to estimate the tissue storage modulus E' and the loss modulus E" using a frequency sweeping method. On the one hand, E' is estimated under a low-frequency (0.1 Hz) rotating magnetic field. In this case, the robot 20 deformation magnitude is only dependent on E' and the effect of E" is negligible.

As illustrated in Fig. 6B, the robot 20 shows distinct deformation magnitudes when interacting with materials 10 of different E', upon applying the same low-frequency magnetic field (24 mT, 0.1 Hz). The average material 10 strain

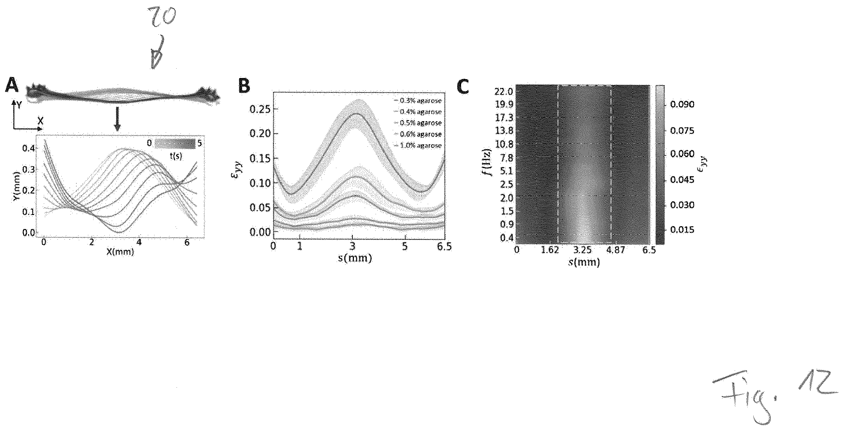

$\overline{\epsilon_{yy}}$ at the robot 20-material 10 interface, calculated by $\overline{\epsilon_{yy}} = \int_{0.3L}^{0.7L} \epsilon_{yy}(s)\,ds\,/0.4L$ (L= 6.5 mm, see Fig. 6B (i) and Fig. 12), varies significantly with the amplitude of 0.33 and 0.18 for E'=1.9 kPa and 3.2 kPa, respectively.

On the other hand, E" is estimated from the robot 20 deformation magnitude change by sweeping the rotating magnetic fields at different frequencies. As shown in Fig. 6C, the robot 20 deforms less for the same material 10 when the actuation frequency increases, and the $\overline{\epsilon_{yy}}$ amplitude drops from 0.56 to 0.36 under the actuation frequency of 3.9 Hz and 14.2 Hz. In the method according to the present invention, the scaling factors k and $k_\tau$, independent of materials 10, are firstly calibrated using synthetic materials 10 to obtain the function E'($\overline{\epsilon_{yy}}$/B$_y$) and E"($\overline{\epsilon_{yy}}$/B$_y$), which are further employed to estimate E' and E" of tissues.

First, E'($\overline{\epsilon_{yy}}$/B$_y$) is estimated under the actuation frequency of 0.1 Hz. In Fig. 6D, agarose and gelatin gels are used as the training and validation materials 10 to obtain the fitted function $\overline{\epsilon_{yy}}$/B$_y$ = 54.128(E')$^{-1.067}$. In this function, k is calibrated as 54.128 Pa/mT, and the index value -1.067 is close to -1, which validates that E" has a negligible effect on the estimation of E' under the 0.1 Hz actuation. It is notable that the E' of the experiment synthetic materials 10 ranges from $1.9 \pm 0.2$ kPa to $51.2 \pm 0.6$ kPa, which covers the stiffness range of most soft tissues.

After E' is estimated, the frequency sweeping method is used to compute the time constant $\tau$, as shown in the subplot of Fig. 6E. Further, $k_\tau$, the ratio of $\tau$ to E"/E', is calibrated with the agarose-based viscoelastic gel and further validated by the gelatin-based gel and chicken breast tissue ($k_\tau$ = 0.0703 sec, Fig. 6E), through which E" is obtained based on the estimated E' and $\tau$.

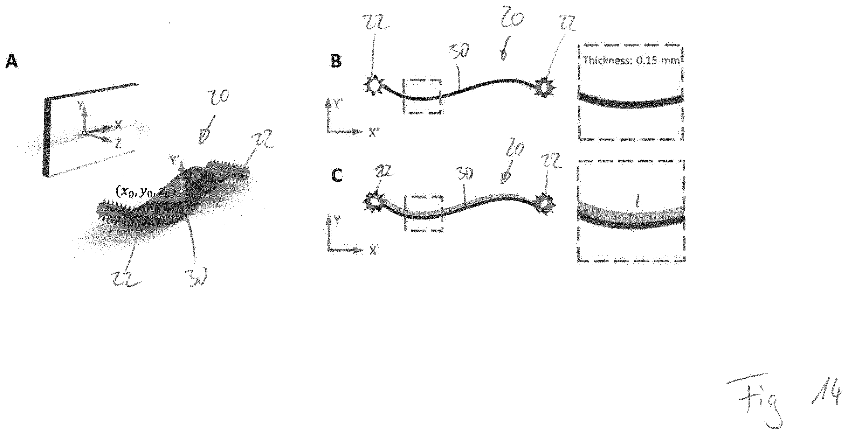

Last, the healthy porcine gastrointestinal (GI) tissues are adopted to test the viscoelasticity probing method ex vivo. Figures 6F and 6G show that the estimated E' and E" of the tissues match the reference moduli characterized by the rheometer. The relative errors are within 8% for E' and 12% for E", as shown in the subplots of Fig. 9C and 7D. Note that the process mentioned above is conducted under the circumstance where the influence of the sample boundary condition on the robot 20 deformation is minimal (see Fig. 13), and the $\overline{\epsilon_{yy}}$ in the robot 20 coordinate system 100 is adopted for the calibration process (see Fig. 14).

The robot 20 according to the present invention also has the potential to achieve distributed probing due to the continuous stress imposed on the contact interface along the robot 20 and the shape changeable member 30, respectively. The method can be potentially used for disease location probing during its development, such as cystic fibrosis in lungs.

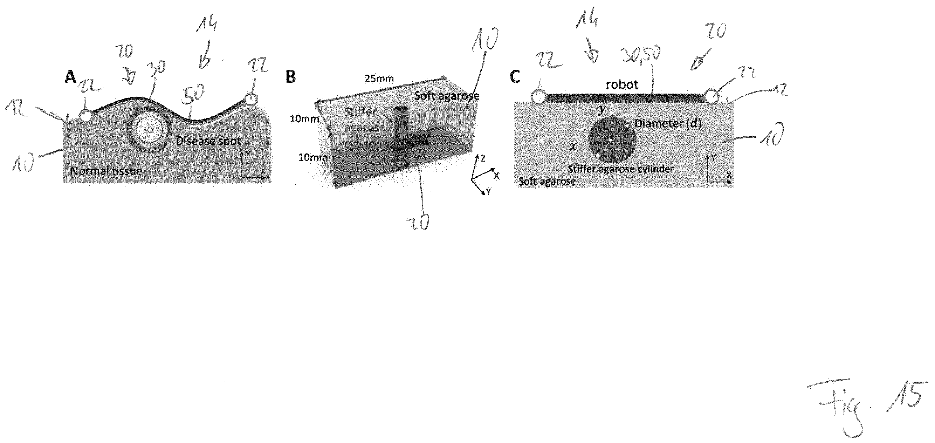

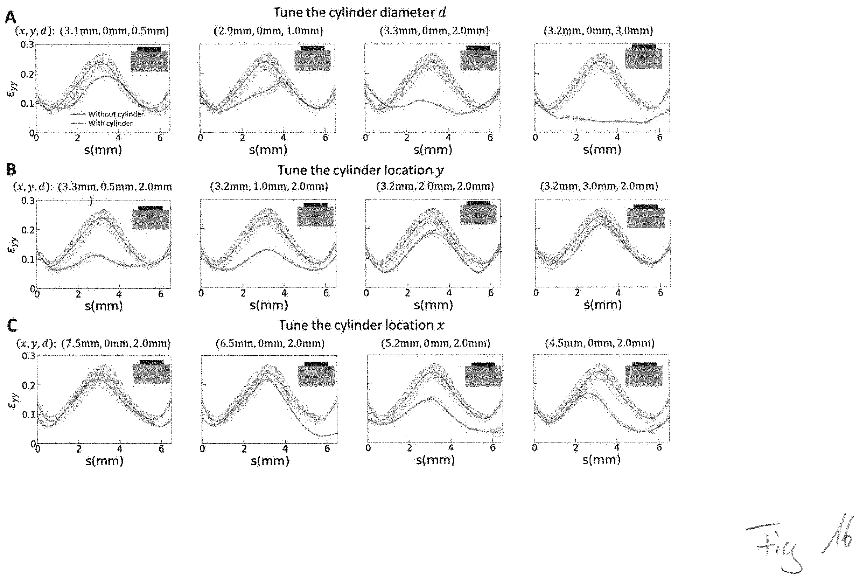

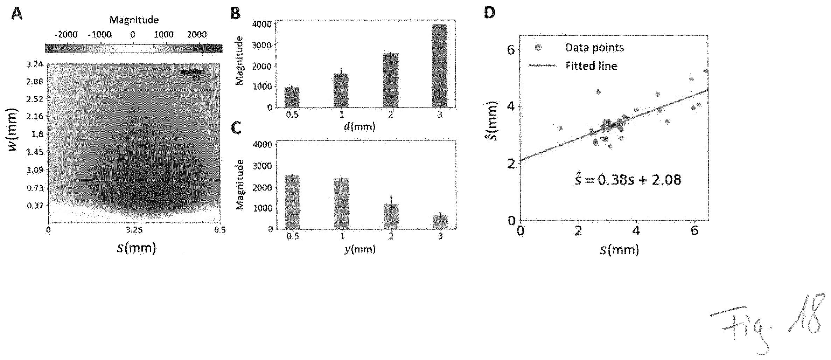

As illustrated in Fig.6 H (i), the robot 20 contacts the stiff cylinder and is actuated by the rotating magnetic field, during which the robot 20 deformation difference with and without a stiff cylinder can indicate the cylinder location in Fig.6 H (ii). Correspondingly, the material 10 deforms less at the cylinder location (Fig.6 H (iii)). In Fig. 15, the location and diameter of the cylinder are adjusted to simulate different occasions, and the corresponding robot 20 and material 10 deformation are shown in Fig. 16 and 15. By analyzing the robot 20 deformation, the cylinder location and its influence on the surrounding area can be quantified (Fig. 18).

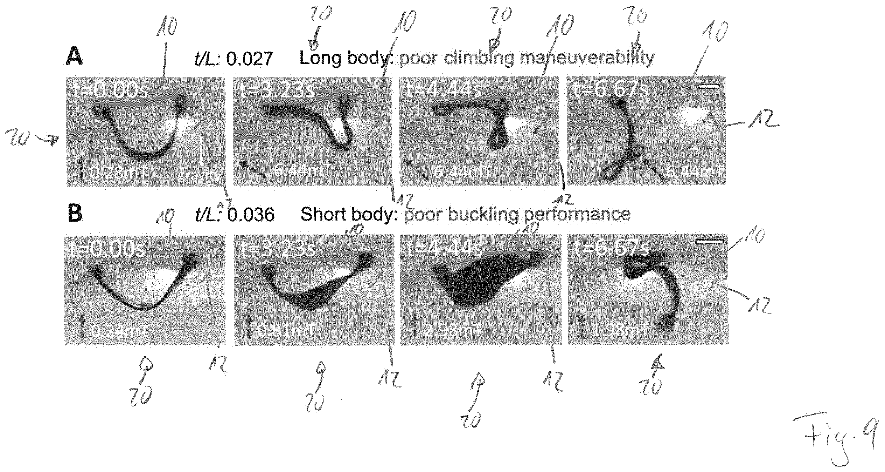

In the following, probing soft tissue mechanics ex vivo for organs with diseases will be described. To show the potential of using the robot 20 according to the present invention to monitor and understand the biomechanics of soft tissues with diseases, we present ex-vivo probing tissue pH and viscoelastic properties of soft tissues using our robot 20 together with X-ray imaging in Fig. 9.

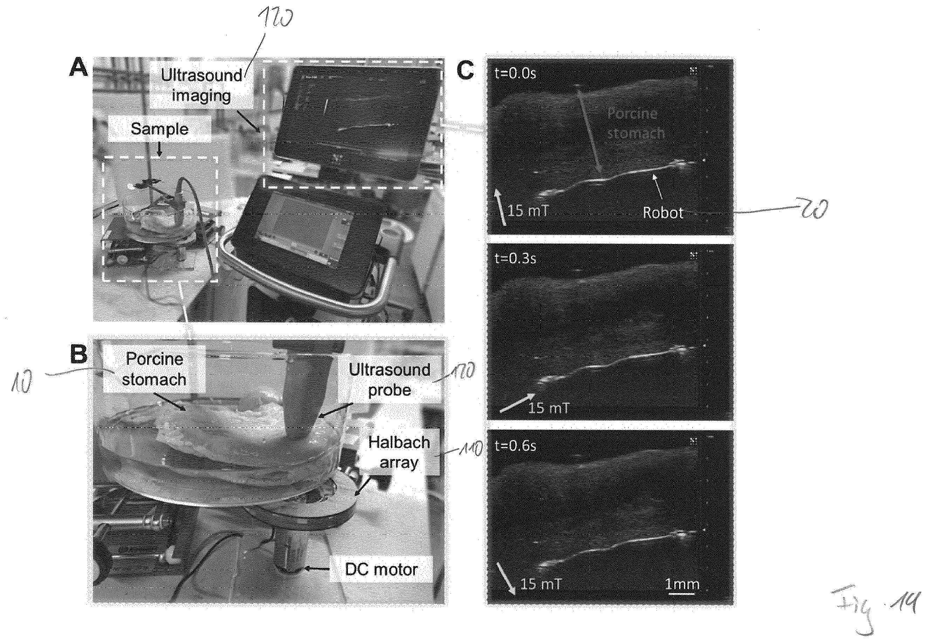

The robot 20 is first deployed into the diseased area of the mouse models and then actuated by the external magnetic field to implement the probing functions. An X-ray cabinet imaging tool tracks the robot 20 and surrounding soft tissues during the probing processes. Figs. 7B, C show that the static, quasi-static and dynamic shapes of the robot 20 and the shape changeable member 30, respectively, can be distinguished from the soft tissues in the X-ray images. For dynamic shape-based probing, this framerate can allow probing the storage modulus E′ of soft materials 10 and the loss modulus E″ of the soft material 10 with a cut-off frequency up to 7 Hz based on the Nyquist sampling criterion. The X-ray imaging device has a frame rate of 30 frames per second (FPS), which is sufficiently fast for imaging robot 20-body dynamic motion with a frequency less than 10 Hz. Notably, the X-ray radiation dose in our experiment is under 410 $\mu$Sv/h, which is safe for live animal experiments. In addition, also ultrasound imaging can be used for tracking the shape and shape change, respectively, of the robot 20 and the shape changeable member 30, respectively, to sense tissue viscoelastic properties with a framerate up to 30 FPS (Fig. 19).

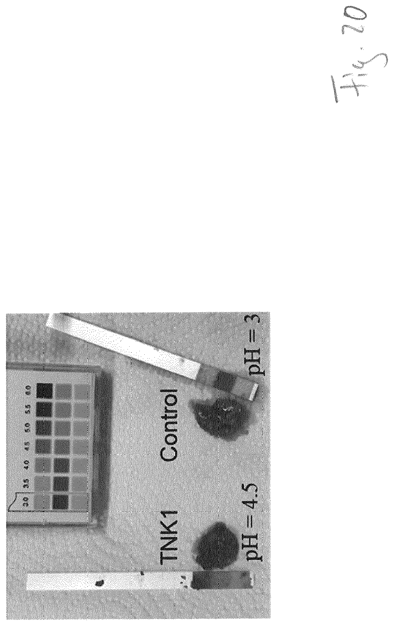

Further, mice with forced expression of thirty-eight-negative kinase 1 (TNK1), which exhibit impaired intestinal barrier and healthy wild type littermates, are used to showcase the probing method according to the present invention for probing pH and viscoelasticity to provide data for subsequently diagnosing diseases. Regulation of apoptosis and immune-modulatory functions have been ascribed to TNK1, which is a promising target during multi-organ dysfunction syndrome (MODS) to prevent damage in several organs, especially the gut. Due to the TNK1 expression, the normal intestinal architecture is perturbed. Additionally, the basal pH in the stomach of the TNK1-expression mouse is higher than that of a mouse from the healthy control group (Fig. 20) since the diseased mice are fasted due to abnormal intestinal activities.

Figure 7D compares the robot 20-tissue adhesion estimated by the probing method according to the present invention, the reference data measured by the high precision force sensor, and the pH value measured by the pH indicator of the mice stomach tissues with (H1 to H5) and without a TNK1-expression related intestinal disorder/disease (D1 to D5). Despite the deviations among individuals from the same group, the TNK1-expression disease shows high relevance with the estimated adhesion results, which also matches the reference results and the corresponding pH values. The proposed adhesion probing method according to the present invention shows its ability to indicate the difference in adhesion or pH of the ex vivo tissues, with 0.32$\pm$0.01 mN to 0.44$\pm$0.01 mN for TNK1-expression diseased mice compared to 0.47$\pm$0.02 mN to 0.59$\pm$0.02 mN for healthy mice, potentially serving as a minimally invasive tool for providing data for subsequently detecting diseases.

Figure 7E compares the storage and loss moduli estimated using the method according to the present invention and the reference data characterized by a rheometer of the colon tissues of mice with (H1 to H5) and without a TNK1-expression related disease (D1 to D5). The estimated storage and loss moduli for the TNK1-expression mice range from 1.9$\pm$0.3 to 3.4$\pm$0.8 kPa and 0.5$\pm$0.1 to 1.2$\pm$0.3 kPa, respectively, whereas the estimated storage and loss moduli for the healthy mice are from 6.5$\pm$1.3 to 11.3$\pm$1.8 kPa and 1.8$\pm$0.4 to 3.8$\pm$0.6 kPa, respectively. The estimated results are consistent with the reference data measured by the rheometer and can indicate the abnormal viscoelastic properties of the tissues.

## General Discussion

We have reported a generic framework to sense the physiological properties of materials, in particular soft tissues, using wireless miniature soft robots comprising shape changeable members 30 and imaging system, in particular medical imaging. A fundamental magnetic-mechanical model is developed to recover the mechanical properties of soft tissues, such as adhesion and viscoelasticity, with the tracked robot shape changes using medical imaging. This framework has been showcased with two example applications. One is a soft robot with an integrated pH-responsive adhesive probing patch for probing robot-tissue adhesion and therein the correlated pH values using a buckling motion. The other one is a soft robot with an integrated adhesive probing patch that can be deployed on soft tissue surfaces and measure the viscoelasticity of soft tissues using a dynamic undulating motion. We have performed systematic experiments on synthetic materials and ex vivo soft tissues and validated the probing mechanisms by comparing them with direct and high-precision measurements of the synthetic materials and biological soft tissues. Finally, we have demonstrated the feasibility of probing the mechanics of soft tissues in mice with diseases as a tool for diagnostics and monitoring GI tract diseases.

Compared with other implantable magnetic and electric devices where the components are embedded inside the tissues, our device can traverse complex soft and wet tissues to provide minimally invasive probing ability to a wide range of tissue properties. Our robots can bond to the surfaces of soft tissues on demand and sense the advanced physiological properties of soft tissues. The deployment of the robot can be realized by a soft-body self-deformation or using an existing medical device, such as an endoscope. On the other hand, existing miniature robots have only shown simple in vitro probing of temperature using thermally responsive liquid elastomer materials on a magnetic body. Our soft robot-based approach can locally sense the tissue biomechanics in vivo, where the medical imaging signals are amplified by the body shapes allowing probing various physiological properties of soft tissues.

Our demonstrated probing mechanisms can potentially be used for probing peptic ulcers in the GI tract, where the pH level changes dramatically compared with normal tissues, as well as pulmonary fibrosis and cancer, where the viscoelastic properties have changed substantially when the disease is developing. The proposed framework of using wirelessly actuated miniature robots to interact with soft tissue surfaces while monitoring their shape deformation using medical imaging thus opens a door for monitoring and understanding tissue biomechanics in vivo during disease development as well as providing feedback information for the applied therapeutic solutions. Our method thus adds unprecedented capabilities to available minimally invasive probing methods and medical devices, with potential for enabling versatile applications in both basic research and clinical practice.

## Materials and Methods

Fabrication of the robot comprising a single sheet-like shape changeable member 30, denoted in the following as robot or soft millirobot. The footpads were made of PDMS (Sylgard 184, Dow Inc.) with a weight ratio of 20:1 between the monomer to the crosslinker using a two-step molding method. A spike mold was prepared using a two-photon polymerization (2PP) 3D printer (Photonic Professional GT, Nanoscribe GmbH) with a rigid commercial photo resin (IP-S, Nanoscribe GmbH). The spike mold has $9 \times 7$ conical spike arrays of 200 $\mu$m in height, 100 $\mu$m in diameter and 200 $\mu$m in spacing, and a 2 mm $\times$ 1.5 mm $\times$ 60 $\mu$m backing layer. We pipetted the benzophenone solution (20 wt% in ethanol, Sigma-Aldrich Inc.) over the micro-spike patch as the hydrophobic photo-initiator and coated the patch with the polyethylene glycol) diacrylate (PEGDA, Sigma-Aldrich, Inc.) as a dissipative hydrogel layer and a chitosan-based bio-adhesive as the tough adhesive for tissue surfaces sequentially. The bio-adhesive for the footpads was prepared by dissolving the chitosan (high molecular weight, Sigma-Aldrich Inc.) as a bridging polymer and the unsulfated N-hydroxysuccinimide (NHS 98%, Sigma-Aldrich Inc.) as a coupling reagent 12 mg/mL into the compound 2-(N-morpholino)ethanesulfonic acid (MES) buffer (Sigma-Aldrich Inc.) at the weight ratio of 2.0% and 0.12%, respectively.

## Fabrication of the adhesive probing patch for probing adhesion and pH.

The adhesive probing patch for probing dry adhesion had a back layer of Ecoflex-0030 silicone rubber. It was cut into a 0.5 mm $\times$ 2 mm $\times$ 100 $\mu$m rectangular sheet prepared using a laser machine (LPKF ProtoLaser U3, LPKF Laser & Electronics AG). The sheet was bonded to the middle part of the robot and the shape changeable member 30, respectively, using uncured Ecoflex-0030 as glue. The adhesive probing patch used for probing tissue adhesion consists of three layers: the 100 $\mu$m thick elastomer, the dissipative hydrogel, and the pH-responsive bio-adhesive. The elastomer layer was prepared the same way as the used dry adhesive probing patch. Then we pipetted the benzophenone solution (20 wt% in ethanol) over the patch as the hydrophobic photo-initiator and coated the patch with PEGDA as a dissipative hydrogel layer. To prepare the pH-responsive bio-adhesive, 5,5',6,6'-tetrahydroxy-3,3,3',3'-tetramethyl-1,1'-spirobiindane (Sigma-Aldrich Inc.), boric acid (Sigma-Aldrich Inc.), and sodium hydroxide (Sigma-Aldrich Inc.) were dissolved into deionized water with a concentration of 0.11 g/mL, 0.022 g/mL, and 0.02 g/mL in the final solution. The mixture was stirred at 90°C for 8 hours for reaction and then filtrated with a 0.2 $\mu$m sterile syringe filter. Polyvinyl alcohol (Mw 89,000-98,000, 99+% hydrolyzed; Sigma-Aldrich Inc.) and the chitosan-based bio-adhesive solution were added to the solution at a weight ratio of 1:3:4 and then stirred at 90°C for 1 hour. The mixture solution (~0.2 $\mu$L) was pipetted over the hydrogel layer of the patch.

## Fabrication of the adhesive probing patch for probing tissue viscoelasticity.

Benzophenone solution (20 wt% in ethanol) was pipetted over the robot and the shape changeable member 30, respectively, to initiate the surface bonding to hydrogels by serving as the hydrophobic photo initiator. PEGDA was poured onto the robot and the shape changeable member 30, respectively, and then degassed for 30 min. After curing in a 365 nm UV chamber (ELG100S, Dinies Technologies GmbH) for 6 min, the coated robot and the shape changeable member 30, respectively, was washed using deionized water to remove the unreacted chemicals. The chitosan-based bio-adhesive was then applied to the robot and the shape changeable member 30, respectively, surface by dip-coating.

**Estimating robot-tissue adhesion.**

The adhesion applied on the robot adhesive probing patch from the tissue surface could be estimated by the static shape of the robot and the shape changeable member 30, respectively, using the Euler-Bernoulli Beam theory. When the adhesive probing patch attaches to the tissue surface, external forces include the three-point contact forces from the tissues (Figs. 4B, C). When the adhesive probing patch detaches from the surface, external forces include only two-point contact forces (Fig. 4D). In both cases, we can use the Euler-Bernoulli Beam model to describe the mechanical behavior of the robot and the shape changeable member 30, respectively. We focus on the first case as we want to estimate the adhesion applied from the tissue surface to the adhesive probing patch, given the static shape of the robot and the shape changeable member 30, respectively, and the external magnetic field. First, for the robot and the shape changeable member 30, respectively, given an infinitesimal element [s, s + ds] (Fig. 4B), the moment balance equation at a static state in Eulerian space is given by,

$$M_b(s + ds) - M_b(s) + \tau_m(s)A_{cross}ds = \left(F_v(s) + \frac{\partial F_v}{\partial s}ds\right)ds\cos\theta - \left(F_h(s) + \frac{\partial F_h}{\partial s}ds\right)ds\sin\theta \, . \, (1)$$

$\tau_m(s)$ is the magnetic torque per volume. $F_h(s)$ and $F_v(s)$ are the internal forces. $M_b(s) = EI\frac{\partial\theta}{\partial s}$ is the bending moment. $A_{cross} = wt$ is the cross area. $\theta(s)$ is the rotation angle, E is the Youngs' modulus, I = tbw/12 is the second moment of the cross-section area (w: width, $t_b$: thickness). Neglecting the high-order terms, we have

$$M_b(s + ds) - M_b(s) + \tau_m(s)A_{cross}ds = F_v(s)ds\cos\theta - F_h(s)ds\sin\theta \, . \qquad (2)$$

The force balance equation is given by

$$-\frac{1}{A_{cross}}\frac{\partial F_h(s,t)}{\partial s} = 0, \qquad (3)$$

$$-\frac{1}{A_{cross}}\frac{\partial F_v(s,t)}{\partial s} = \rho g \, . \qquad (4)$$

If we assume the gravity of an infinitesimal element is negligible compared with the adhesion during the detachment, then we have

$$F_v(s) = F_{1y}, \, s \in \left[0, \frac{L}{2} - \frac{L_0}{2}\right], \, F_v(s) = F_{2y}, \, s \in \left[\frac{L}{2} + \frac{L_0}{2}, L\right], \qquad (5)$$

$$F_h(s) = F_{1x}, \, s \in \left[0, \frac{L}{2} - \frac{L_0}{2}\right], \, F_h(s) = F_{2x}, \, s \in \left[\frac{L}{2} + \frac{L_0}{2}, L\right] \, . \qquad (6)$$

L and $L_0$ are the robot length and adhesive probing patch length, respectively. We integrate Eqn. (2) over $s \in \left[0, \frac{L}{2} - \frac{L_0}{2}\right]$ and $s \in \left[\frac{L}{2} + \frac{L_0}{2}, L\right]$ and have Eqns. (7) and (8), respectively.

$$\int_0^{s_l} -\frac{\partial M_b(s)}{\partial s} ds = \int_0^{s_l} \tau_m(s) A_{cross} ds - \int_0^{s_l} F_v(s) \cos\theta ds + \int_0^{s_l} F_h(s) \sin\theta \, ds$$

$$\Rightarrow -M_b(s_l) + M_b(0) = \tau_{net,l}(s_l) - F_{1y}\int_0^{s_l} \cos\theta ds + F_{1x}\int_0^{s_l} \sin\theta ds$$

$$\Rightarrow M_b(0) - M_b(s_l) = \tau_{net,l}(s_l) + F_{1y}d_{1x}(s_l) - F_{1x}d_{1y}(s_l) \tag{7}$$

$d_{1x}(s_l)$, $d_{1y}(s_l)$ are the x and y components of the distance between the robot foot pad 1 and the position $s_l$ when adhering to the tissue surface. $\tau_{net,l}(s_l) = \int_0^{s_l} \tau_m(s) A_{cross} ds$ .

$$\int_{s_r}^{L} -\frac{\partial M_b(s)}{\partial s} ds = \int_{s_r}^{L} \tau_m(s) A_{cross} ds - \int_{s_r}^{L} F_v(s) \cos\theta ds + \int_{s_r}^{L} F_h(s) \sin\theta \, ds$$

$$\Rightarrow -M_b(L) + M_b(s_r) = \tau_{net,r}(s_r) - F_{2y}\int_{s_r}^{L} \cos\theta ds + F_{2x}\int_{s_r}^{L} \sin\theta \, ds$$

$$\Rightarrow M_b(s_r) - M_b(L) = \tau_{net,r}(s_r) + F_{2y}d_{2x}(s_r) - F_{2x}d_{2y}(s_r) \tag{8}$$

$d_{2x}(s_r)$, $d_{2y}(s_r)$ are the x and y components of the distance between the position $s_r$ and the robot foot pad 2 when adhering to the tissue surface. $\tau_{net,r}(s_r) = \int_{s_r}^{L} \tau_m(s) A_{cross} ds$ .

Based on the pinned-pined boundary conditions at the two ends of the robot and the shape changeable member 30, respectively, we have

$$\frac{\partial\theta}{\partial s}(0) = 0, \tag{9}$$

$$\frac{\partial\theta}{\partial s}(L) = 0. \tag{10}$$

From Eqns. (7-10) we have

$$-M_b(s_l) = \tau_{net,l}(s_l) + F_{1y}d_{1x}(s_l) - F_{1x}d_{1y}(s_l), \tag{11}$$

$$M_b(s_r) = \tau_{net,r}(s_r) + F_{2y}d_{2x}(s_r) - F_{2x}d_{2y}(s_r). \tag{12}$$

According to the static shape of the robot and the shape changeable member 30, respectively, $\theta(s), \frac{\partial\theta}{\partial s}$ , $\tau_{net,l}(s_l)$, $\tau_{net,r}(s_r)$, $M_b(s_l)$, $M_b(s_r)$ could be obtained. By adjusting $s_l$, $s_r$ with a set of values $\{s_{l,1}, s_{l,2} \cdots, s_{l,m}\}$ and $\{s_{r,1}, s_{r,2} \cdots, s_{r,k}\}$, multiple equations of (11) and (12) can be obtained, which can be written in the following matrix form:

$$\begin{bmatrix} -d_{1y}(s_{l,1}) & d_{1x}(s_{l,1}) \\ -d_{1y}(s_{l,2}) & d_{1x}(s_{l,2}) \\ -d_{1y}(s_{l,3}) & d_{1x}(s_{l,3}) \\ \vdots & \vdots \\ -d_{1y}(s_{l,m}) & d_{1x}(s_{l,m}) \end{bmatrix} \begin{bmatrix} F_{1x} \\ F_{1y} \end{bmatrix} = \begin{bmatrix} -M_b(s_{l,1}) - \tau_{net,l}(s_{l,1}) \\ -M_b(s_{l,2}) - \tau_{net,l}(s_{l,2}) \\ -M_b(s_{l,3}) - \tau_{net,l}(s_{l,3}) \\ \vdots \\ -M_b(s_{l,m}) - \tau_{net,l}(s_{l,m}) \end{bmatrix} \qquad (13)$$

$$\begin{bmatrix} -d_{2y}(s_{r,1}) & d_{2x}(s_{r,1}) \\ -d_{2y}(s_{r,2}) & d_{2x}(s_{r,2}) \\ -d_{2y}(s_{r,3}) & d_{2x}(s_{r,3}) \\ \vdots & \vdots \\ -d_{2y}(s_{r,k}) & d_{2x}(s_{r,k}) \end{bmatrix} \begin{bmatrix} F_{2x} \\ F_{2y} \end{bmatrix} = \begin{bmatrix} M_b(s_{r,1}) - \tau_{net,r}(s_{r,1}) \\ M_b(s_{r,2}) - \tau_{net,r}(s_{r,2}) \\ M_b(s_{r,3}) - \tau_{net,r}(s_{r,3}) \\ \vdots \\ M_b(s_{r,k}) - \tau_{net,r}(s_{r,k}) \end{bmatrix}, \qquad (14)$$

where the subscript (m) and (k) represent the m-th value of $s_l$ and k-th value of $s_r$. Eqns. (13) and (14) can be represented as

$$D_1 f_1 = M_1 , \qquad (15)$$

$$D_2 f_2 = M_2 . \qquad (16)$$

Here, $D_1$, $f_1$, $M_1$, $D_2$, $f_2$, $M_2$ represent the corresponding matrix and vector in (13) and (14).
Subsequently, $f_1$ and $f_2$ can be solved by employing the following optimization process:

$$\text{minimize} \quad (D_1 f_1 - M_1)^T Q_1 (D_1 f_1 - M_1), \qquad (17)$$

$$\text{minimize} \quad (D_2 f_2 - M_2)^T Q_2 (D_2 f_2 - M_2), \qquad (18)$$

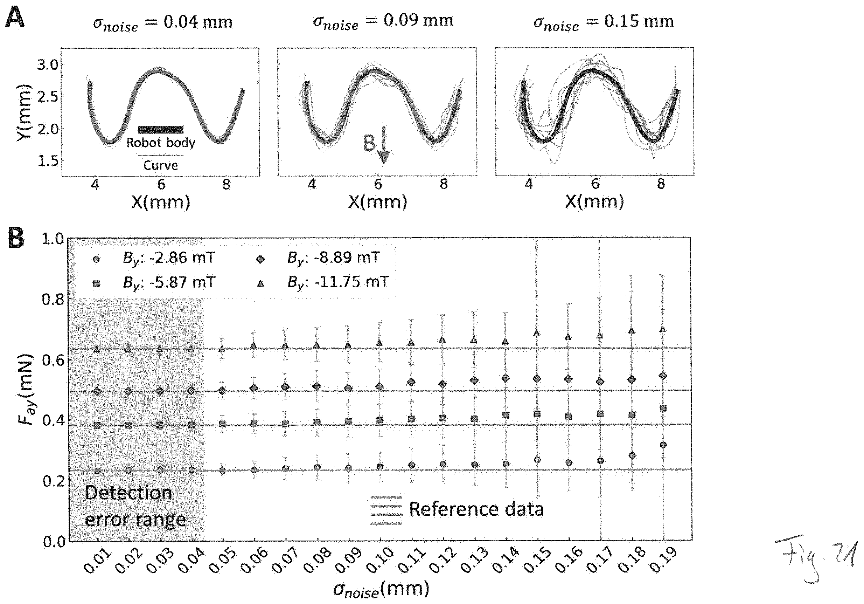

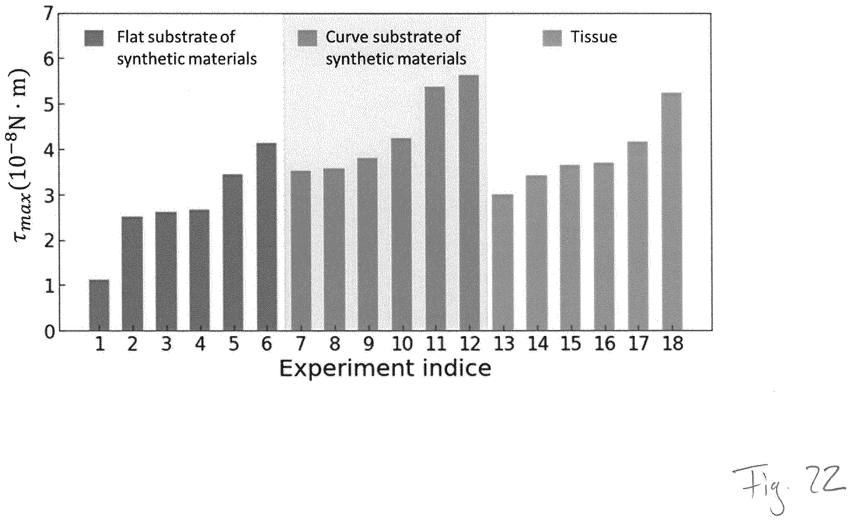

where $Q_1$ and $Q_2$ are the matrices that give higher weightings to the robot and the shape changeable member 30, respectively, positions considered more important. The optimization process can be solved with solvers, such as quadratic optimization, which can minimize the effect of the detection noise, as shown in Fig. 21. Furthermore, we assume the reaction torque at the patch-substrate interface is negligible, as shown in Fig. 22. For the whole robot and the shape changeable member 30, respectively, we have the force balancing equations

$$F_{1x} + F_{2x} + F_{ax} = 0, \qquad (19)$$

$$F_{1y} + F_{2y} + F_{ay} = F_g , \qquad (20)$$

where $F_g$ is the gravity of the bio-adhesive probing patch. Therefore, with Eqns. (17-20), we could estimate $F_{ax}$ and $F_{ay}$.

**Estimating tissue viscoelastic properties.**

We use the following viscoelastic constitutive equation to model the soft tissues.

$$\sigma^t = \mu_t J_t^{-\frac{5}{3}} \left( F_t F_t^T - \frac{I_1}{3} I \right) + K_t (J_t - 1) I, \quad \Omega_t \times T \qquad (21)$$

where $\sigma^t$ is the Cauchy stress of the soft material, $\mu_t$ and $K_t$ are the material's shear modulus and bulk modulus,

respectively. $F_t$ represents the deformation gradient tensor. $I_1 = \text{tr}(FF^T)$ is the first invariant of the left Cauchy-Green tensor. The quantity $J_t$ is the volumetric Jacobian of the deformation, as defined as $J_t = \det F_t$. $\Omega_t$ is the domain of the soft tissue. T is the domain of time. For an incompressible solid, the deformation satisfies $J_t = 1$. We have the stress and strain relationship given by

$$\sigma^t = C_t \epsilon^t + \eta_t \frac{d\epsilon^t}{dt}, \qquad \Omega_t \times T \qquad (22)$$

where $\epsilon^t$ is the strain of the soft materials, $C_t$ is the elasticity tensor, and $\eta_t$ is the viscosity of the soft material. The normal stress-strain relationship of the soft material is given by

$$\sigma_{yy}^t = E_t \epsilon_{yy}^t + \eta_t \frac{d\epsilon_{yy}^t}{dt}. \qquad (23)$$

We propose a frequency-sweeping method to estimate $E_t$ and $\eta_t$. By representing the system states in the frequency domain, we have

$$\sigma_{yy}^t(j\omega) = (E_t + \eta_t j\omega)\epsilon_{yy}^t(j\omega), \qquad (24)$$

$$\frac{\epsilon_{yy}^t(j\omega)}{\sigma_{yy}^t(j\omega)} = \frac{1}{j\eta_t\omega + E_t} = \frac{1}{E_t} \cdot \frac{1}{j\frac{\eta_t}{E_t}\omega + 1}, \qquad (25)$$

where $\omega$ is the frequency and j is the imaginary unit. Similarly, the soft robot could also be modeled following the generalized neo Hookean model for magnetic composite materials (39), given by

$$\sigma^r = \mu_r J_r^{-\frac{5}{3}}\left(F_r F_r^T - \frac{I_{1r}}{3}I\right) + K_r(J_r - 1)I - \frac{1}{J}B \otimes F_r M, \; \Omega_r \times T, \qquad (26)$$

$$\sigma^r = C^r \epsilon^r, \quad \Omega_r \times T \qquad (27)$$

where $\sigma^r$ is the Cauchy stress of the robot, where $\sigma^r$ is the Cauchy stress of the robot soft body, $\mu_r$ and $K_r$ are the material's shear modulus and bulk modulus, respectively. $F_t$ represents the deformation gradient tensor. $I_{1r} = \text{tr}(F_r F_r^T)$ is the first invariant of the left Cauchy-Green tensor. The quantity $J_r$ is the volumetric Jacobian of the deformation, as defined as $J_r = \det F_r$. $\Omega_r$ is the domain of the soft tissue. We further have the boundary conditions given by

$$\sigma^r n = -\sigma^t n, \quad \partial\Omega_{rt} \times T, \qquad (28)$$

where n is the tissue surface normal unit vector. Given a specific robot, we assume that the normal stress of the soft tissue is proportional to the external magnetic field with a scaling factor, which could be calibrated. $k_y$ is a function of the bulk modulus $K_r$ and the magnetization 32 profile M(s) of the robot and independent from the external magnetic field and tissues. $\epsilon_{yy}^t$ can be estimated from the displacement of the tissue at the boundary, $u_y^t = u_y^r$, where $u_y^r$ and $u_y^t$ are the robot and the shape changeable member 30, respectively, displacement and tissue displacement. Based on the Hertzian contact theory, the relation between the strain field distribution $\epsilon_{yy}^t$ and the robot displacement uy can be assumed as the line contact on a half-plane,

$$\epsilon_{yy}^t = -\frac{2y^3}{\pi E_t}\int_a^b \frac{cE_t u_y^r(x')dx'}{[(x-x')^2+y^2]^2} = -\frac{2cy^3}{\pi}\int_a^b \frac{u_y^r(x')dx'}{[(x-x')^2+y^2]^2}, \qquad (29)$$

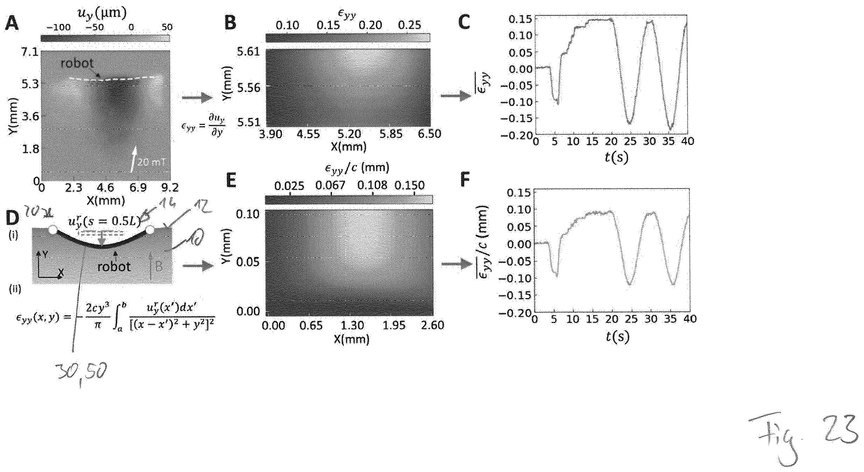

where c is the constant that can be determined by regression, as shown in Fig. 23.

Now we have

$$\frac{\epsilon_{yy}^t(j\omega)}{B_m(j\omega)} = \frac{k_y}{j\eta_t\omega+E_t} = \frac{k_y}{E_t}\cdot\frac{1}{j\frac{\eta_t}{E_t}\omega+1}. \qquad (30)$$

Notably, the elasticity $E_t$ and the viscosity $\eta_t$ in the viscoelastic model represent the same physical terms as the storage modulus $E'$ and the loss modulus $E''$ as measured by a rheometer. Considering this, we correlate the elasticity $E_t$ with the storage modulus $E'$ as $E_t = k_e E'$. Equation 30 is rewritten as

$$\frac{\epsilon_{yy}^t(j\omega)}{B_m(j\omega)} = \frac{k_y}{k_e E'}\cdot\frac{1}{j\frac{\eta_t}{E_t}\omega+1} = \frac{k}{E'}\cdot\frac{1}{j\frac{\eta_t}{E_t}\omega+1}. \qquad (31)$$

Here, we define $k = \dfrac{k_y}{k_e}$, which was calibrated by the regression shown in Fig. 6D.

We correlate the time constant $\tau = \dfrac{\eta_t}{E_t}$ with the ratio $\dfrac{E''}{E'}$ as $\tau = \dfrac{\eta_t}{E_t} = k_\tau \dfrac{E''}{E'}$. Equation (31) can be expressed as

$$\frac{\epsilon_{yy}^t(j\omega)}{B_m(j\omega)} = \frac{k}{E'}\cdot\frac{1}{j\frac{\eta_t}{E_t}\omega+1} = \frac{k}{E'}\cdot\frac{1}{j\left(\frac{k_\tau E''}{E'}\right)\omega+1} \qquad (32)$$

where the coefficient $k_\tau$ was calibrated by regression, as illustrated in Fig. 6E.

**Preparation of synthetic materials for adhesion measurements.**

The synthetic materials used for adhesion characterization included Dragon Skin 0020 (Smooth-On Inc.), Dragon Skin FxPro (Smooth-On Inc.), and Ecoflex 0030 (Smooth-On Inc.). The corresponding monomer and cross-linker were mixed at the ratio denoted as the prefix of the name of the surface and degassed for 5 min. The mixture of two milliliters was then poured into a small petri dish and cured at 60°C for 10 min. A 2 mm thick rectangular sheet (size: 10 mm × 5 mm) was cut for adhesion tests. Additionally, the 3D phantom models with surfaces of various curvatures were prepared using a 3D printer (Form 3, Formlabs, Inc.) with the photo resin Clear V4 (Formlabs, Inc.). Then the Ecoflex 0030 rubber was applied to the surfaces by dip-coating. The tested materials included Ecoflex 0030 with a weight ratio of 1:1 and 2:1, Dragon Skin FxPro with a weight ratio of 1:2 and 2:1, and Dragon Skin 0020 with a weight ratio of 1:1.

**Preparation of synthetic materials for viscoelasticity measurements.**

The agarose gel samples were prepared in the following steps. First, agarose powder (BioReagents™ A9539, Sigma-Aldrich Co.) was mixed with deionized water, after which the mixture was continuously heated and stirred at 90°C until all the powder dissolved. Then, the solution was boiled for another 5 minutes, poured into a 3D-printed container (photo resin Clear V4, Formlabs, Inc.) with a dimension of 25 mm × 10 mm × 10 mm (length × width × thickness), and then cooled at room temperature 24°C for about 30 minutes. Subsequently, the robot with the bio-adhesive was attached to the gel. The same procedure was repeated for preparing agarose gel samples with varying thicknesses using different containers. The agarose gels with the weight ratio of 0.3 wt%, 0.4 wt%, 0.5 wt%, 0.6 wt% and 1.0 wt% were prepared. The gelatin powder from porcine skin (-300 g Bloom, Type A, Sigma-Aldrich Co.) was mixed with deionized water and then stirred at 90°C. After the gelatin powder fully dissolved, the solution was cooled down at 60°C for 20 minutes and congealed when immersed in ice water. Thereafter, the robot was attached to the gelatin gel. The gelatin gels with the weight ratio of 3.0 wt%, 4.0 wt% and 5.0 wt% were prepared.

Sucrose was used for the preparation of viscoelastic samples. The sucrose powder (14378, Sigma-Aldrich Co.)

was added to the deionized water, and the mixture was heated and stirred at 90°C until all the powder dissolved. Then, the gels were made through the same procedures above. For example, 60-0.3 agarose-based gel (weight ratio, sucrose: water: agarose - 60: 40: 0.3) was used for testing the material viscoelasticity in Fig. 6C. Likewise, 50-0.3, 60-0.2, 60-0.4, 70-0.3 agarose-based gels, and 50-3 and 60-3 gelatin-based gels were used. Fluorescent red polyethylene microspheres (UVPMS-BR-1.090 27-32 μm, Cospheric Co.) were adopted to visualize the deformation of the material under a fluorescent microscope (Leica M165 FC, Leica Microsystems). The mixture of the fluorescent microspheres (diameter: 27-32 μm) and surfactants Polysorbate 80 (P1754, Sigma-Aldrich Co.) was added to the agarose gel solution and stirred at room temperature for at least 2 minutes, after which the mixture was cooled for more than 30 minutes. The soft agarose gel sample with a rigid cylinder embedded was prepared using the following steps. First, the 1.0 wt% agarose gel solution was poured into the negative cylinder mold, whose one end was sealed by plastic tape. Then, the tape was removed, and the cured cylinder was extracted and placed on the bottom of the container. Afterward, the 0.3 wt% agarose gel solution was poured into it, and the mixture cooled at about 24°C for more than 30 minutes. If the cylinder needed to be at a distance from the robot, the agarose gel with a given thickness was attached to the cylinder side with the 0.3 wt% agarose gel solution. Finally, the robot was attached to the desired position with the abovemen-tioned method.

**Adhesion measurements.**

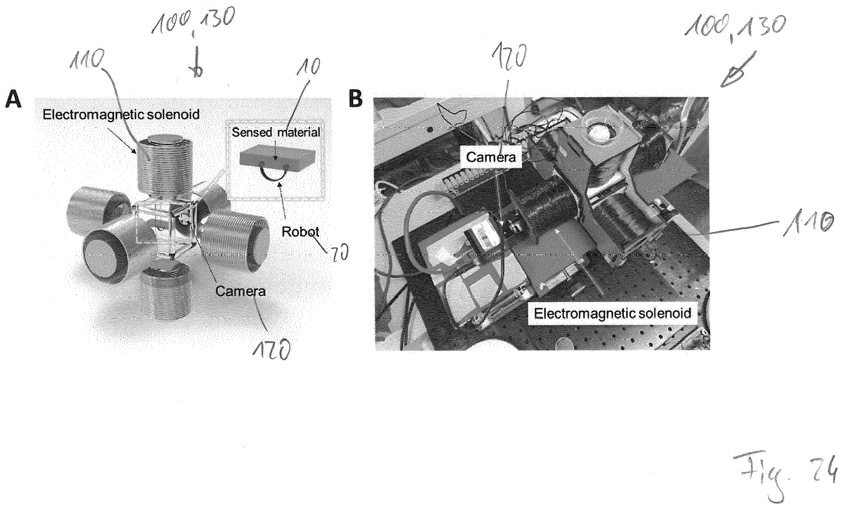

The adhesion measurement using the robot was conducted in the electromagnetic actuation setup, as shown in Fig. 24. To make the measurement consistent for each test, a sequence of external magnetic field waveforms was designed and pre-programmed using LabVIEW (National Instrument, Inc.). As shown in Fig. 4D, the procedures include three steps. First, we increased the magnetic field $\mathbf{B_y}$ from 0 mT to 10 mT rapidly to load the adhesive probing patch for attachment (snapshot at a time interval of 2.5 sec) and maintains for 5 sec. Second, we increased the magnetic field $\mathbf{B_y}$ in a step-wise waveform from 0 mT up to 20 mT in the negative y direction with an interval of 2 mT while maintaining the magnetic field value at each step for 2.5 sec to detach the adhesive probing patch (snapshot at a time interval of 14.2 sec). Lastly, the measurement cycle was completed until the adhesive probing patch was completely detached from the tissue (snapshot at a time interval of 17.3 sec). The procedures were repeated for at least 5 times to reduce the measurement errors.

The adhesion measurements to obtain ground-truth data were conducted in a customized experimental setup. The adhesive probing patches, including the dry adhesive probing patch and the pH-responsive adhesive probing patch, prepared as described above, were cropped into 1 mm × 1 mm square sheets. They were attached to a 3D-printed tip and aligned to the substrate, as shown in Fig. 7B. The tissue samples were prepared for every 10 tests (within 5 min) from the fresh animal organs to avoid the dehydration of the tissues. The approaching and retracting speeds of the probe were both set to 20 μm/s. The preload and contact time were set to 0.5 mN and 3 sec, respectively.

**Viscoelasticity measurements.**

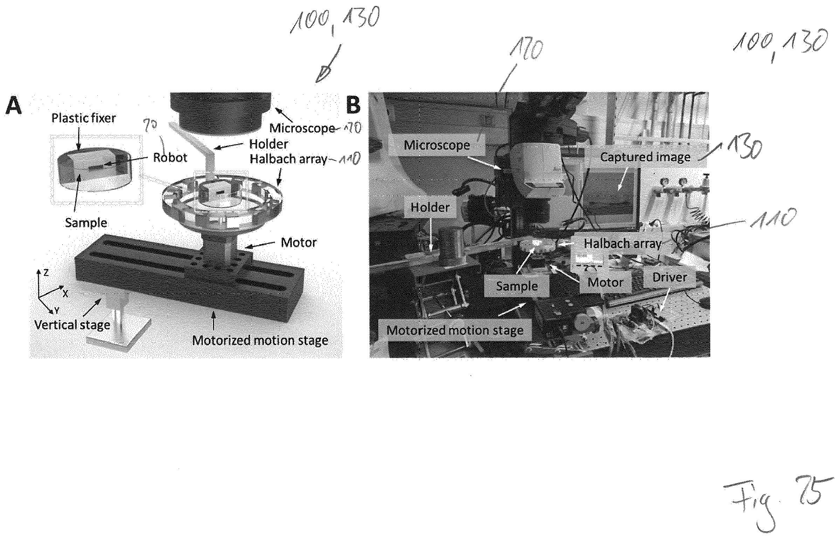

The soft robot attached to a tissue sample was actuated by a rotating magnetic field of different frequencies generated by the Halbach arrays. Four Halbach arrays made of NdFeB N42 grade magnet cubes (W-05-N, W-07-N and W-10-N, Webcraft GmbH) were prepared with a magnitude of 5.8 mT, 12.3mT, 20.1 mT and 24.2 mT in the x-y plane at the center as shown in Fig. 25. A translational motorized stage (LTS300/M, Thorlabs Inc) mounted on the vertical stage (DIN 12897, Bochem Instrumente GmbH) was used to control the position of the Halbach array in the x and z axes, while a stepper motor (535-0372, RS Components GmbH) or DC motor (242478, Maxon Co.) was employed to rotate the Halbach array, as shown in Fig. 25.

To measure the viscoelasticity of the material, the prepared sample was firstly placed under the field-of-view of the microscope. The motorized stage moved the Halbach array to ensure that the robot was located at the center of the array. Then, the motor started rotating at a given frequency. The Halbach array was rotated at 0.1 Hz for probing the material elasticity. Subsequently, the robot was actuated by sweeping the magnetic field from 0.3 Hz to 15 Hz to measure the material viscoelasticity. The magnetic field magnitude was adjusted by using different Halbach arrays and changing the z distance from the robot to the array. The robot showed sufficient large deformation for visualization while not detaching from the material. The whole process was recorded by a high-speed camera mmDK-2740, Dantec Dynamics A/S Inc.) at a sampling rate of 400 FPS. Five material samples were prepared for each type of material. Five soft robot samples were used for each material sample. The viscoelasticity of the samples was characterized using the oscillation frequency module on a rheometer (Discovery HR-2, TA Instruments). The constant strain was set to 0.1 %. The frequency varied from 0.1 to 100 rad/s with 5 test points between 1 and 10 in a log scale. The gel and tissue samples were prepared as 2 mm-thick circular plates with a diameter of 20 mm. The storage and loss moduli used as the ground truth data for quantifying the probing accuracy of our soft robot-based method are the mean values of those measured at the frequency of 0.1 Hz.

**Robot shape tracking and analysis.**

The robot shape tracking was achieved by extracting its edge or segmenting the image plus centerline extraction at each frame, during which the robot footpads were also tracked. A range of discrete points were sampled at the same interval from the extracted centerline or edge and further used to fit the B-spline to represent the robot curve. All the procedures were accomplished by a customized python code, which is available on GitHub (https://github.com/wchunxiang/biomechanics-estimation).

**Visualization and analysis of surface material deformation.**

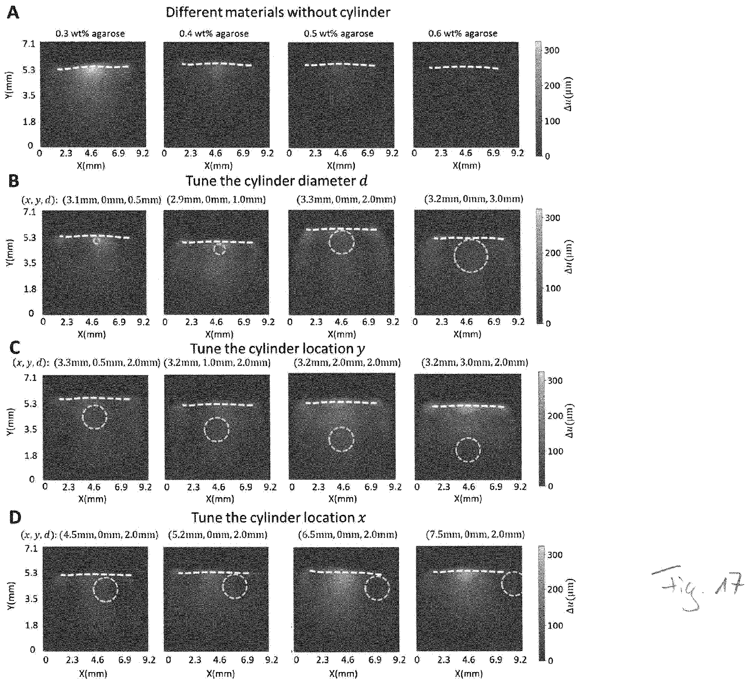

Digital image correlation (DIC) implemented with a customized python code was utilized to compute the displacement field of the material, whose deformation was visualized by fluorescent particles. Based on the assumption that there was no flip between the robot and the shape changeable member 30, respectively, and the tested material, the displacement of the tracked particles and the robot were combined to compute the strain field. The displacement in the x and y axis of the point $\left(x_0^{(i)}, y_0^{(i)}\right)$ in the displacement field were calculated by $u_x\left(x_0^{(i)}, y_0^{(i)}, t=j\right) = x_j^{(i)} - x_0^{(i)}$ and $u_y\left(x_0^{(i)}, y_0^{(i)}, t=j\right) = y_j^{(i)} - y_0^{(i)}$, where $\left(x_j^{(i)}, y_j^{(i)}\right)$ was the position of the i-th particle or robot curve point in the j-th frame and $\left(x_0^{(i)}, y_0^{(i)}\right)$ was the original position of the tracked particle or robot curve point with no magnetic field actuation. Further, the discrete field points were processed by linear interpolation and data smoothing to obtain the displacement field, as shown in Fig. 23. In Fig. 6H and Fig. 17, the value of the maximum displacement field $\Delta u(x,y)$ at position (x,y) was calculated by

$$\Delta u(x, y) = \sqrt{\left(\max\left(u_x(x, y, t)\right) - \min\left(u_x(x, y, t)\right)\right)^2 + \left(\max\left(u_y(x, y, t)\right) - \min\left(u_y(x, y, t)\right)\right)^2}. \quad (33)$$

The wavelet transform used for the analysis of the effect of the cylinder inside the homogeneous bulk material was based on the default function cwt (Continuous 1-D wavelet transform) of the SciPy package (42).

**Correction of the robot curve misalignment for viscoelasticity probing.**

As illustrated in Fig. 14, the error in the extracted robot shape due to the misalignment between the imaging coordinate system xyz and the robot-tissue coordinate system x'y'z' about the x axis can be corrected using the following equation,

$$\begin{bmatrix} x \\ y \\ z \\ 1 \end{bmatrix} = \begin{bmatrix} 1 & 0 & 0 & x_0 \\ 0 & \cos(\varphi) & -\sin(\varphi) & y_0 \\ 0 & \sin(\varphi) & \cos(\varphi) & z_0 \\ 0 & 0 & 0 & 1 \end{bmatrix} \begin{bmatrix} x' \\ y' \\ z' \\ 1 \end{bmatrix} \quad (34)$$

where $(x_0, y_0, z_0)$ is the origin coordinate of x'y'z' in xyz and $\varphi$ is the misalignment angle about the x axis, $\varphi = \arcsin[(l - t)/w]$. Here, l is the nominal thickness of the robot, and t and w are the real thickness and width of the robot and the shape changeable member 30, respectively. Assuming the robot only deforms in the x'y' plane during the actuation, the real robot displacement $u_y'$ can be obtained using the correction, $u_y' = u_y/\cos(\varphi)$, where $u_y$ is the detected robot displacement in the imaging coordinate system.

**X-ray medical imaging.**

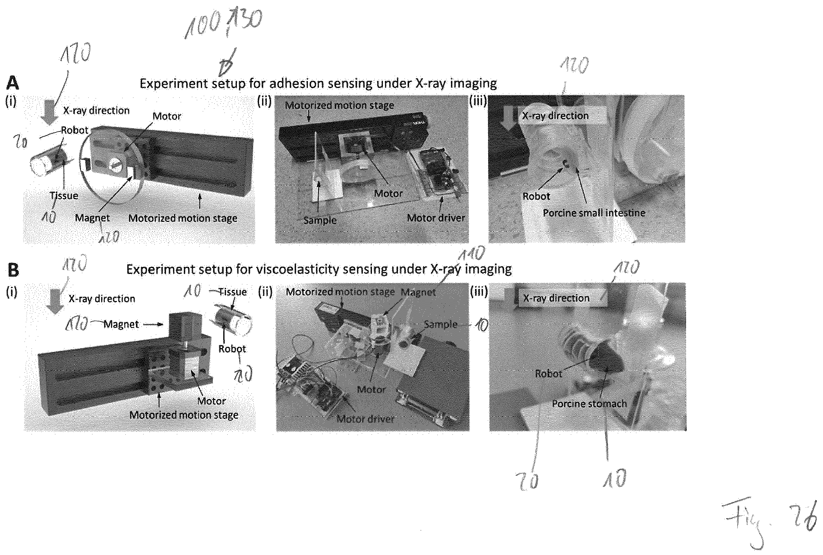

An X-ray imaging device (XPERT 80, KUBTEC, Stratford CT) was used to visualize the robot shape changes on tissues. As illustrated in Fig. 26A, probing adhesion under X-ray imaging was achieved by adjusting the position and orientation of the permanent magnet. With the Halbach array mounted on a rotary motion stage in Fig. 26B, it was possible to demonstrate the robot motion for viscoelasticity probing. The X-ray accelerating voltage was set to 65 kV during the experiments, and the video was recorded at 30 FPS. The X-ray dose was measured with the radiation meter (RM-400, Voltcraft GmbH) under the $\alpha+\gamma+\beta$ mode for the X-ray accelerating voltage 65 kV and current 86 $\mu$A.

**Preparation of animal organs.**

The porcine organs in the GI tract, including the small intestine and stomach and chicken breast, were from the local food factory in Stuttgart, Germany. The rat stomachs were from the Anatomy and Cell Biology Lab in University of Ulm, Germany. The organs were minimally cleaned by emptying the organ while minimizing the surface damage. The tissues used for tests and characterization were within 24 hours and stored in a refrigerator at 2 °C. The organs were cut into blocks with a size of 25 mm $\times$ 15 mm (width $\times$ height) and a thickness of 6.4 $\pm$ 1.4 mm. Then, the robot was attached to the surface to sense viscoelasticity. For viscoelasticity characterization, the organs were cut into circular plates with a radius of 10 mm and a thickness of 5 mm. For the adhesion tests, the organs were cut and attached to a glass slide. Then the robot was put on the tissue to test the adhesion property.

**Preparation of mice with TNK1-expression-related intestinal disorder or disease model.**

The expression of TNK1 in 8-week-old Tnk1-knockin mice (Rosa26rtTA/+, Hprt MycTnk1tg) was induced by the single intraperitoneal injection of doxycycline (50 $\mu$g/g). The control group of healthy mice was treated with the intraperitoneal injection of the same volume of saline solution (0.9% NaCl). Twenty-four hours after the injection, the mice were sacrificed and dissected. Genetic modification of TNK1-knockin mice was verified by genotyping. To prepare the tissues for the viscoelasticity tests, the colon, and the intestinal organs behind it were cut out, and the robot was attached to the internal surface of the opened colon. To prepare the tissues for the adhesion tests, the stomach was opened and minimally cleaned by emptying the inner stuff. All tests were completed within 3 hours.

List of references

| 10 | Material |
|----|----------|
| 12 | Surface |
| 14 | Probing spot |

| 20 | Robot |
|----|-------|
| 22 | Footpad |
| 24 | Body |
| 26 | Microspike |

| 30 | Shape changeable member |
|----|-------------------------|
| 32 | Magnetization |
| 34 | Sheet |
| 36 | Length |
| 38 | Width |
| 40 | Height |

| 50 | Probing patch |
|----|---------------|

| 60 | Elastomer |
|----|-----------|
| 62 | Hydrogel |
| 64 | Bio-adhesive |

| 100 | System |
|-----|--------|

110     Magnetic actuation system
120     Imaging system
130     Data analyzing system

**Claims**

1.  Method for probing one or more properties of a material (10) at a probing spot (14) at the surface (12) of the material (10) by an untethered robot (20), the robot (20) comprising a shape changeable member (30) actuatable by temporally and/or spatially variable magnetic fields,
    **characterized by** the following steps

    a) Providing a temporally and/or spatially variable first magnetic field by a magnetic actuation system (110) for actuating the shape changeable member (30) for creating one or more types of locomotion of the robot (20), and guiding the robot (20) to a probing spot (14) of the material (10) to be probed by accordingly altering the first magnetic field,
    b) Providing a temporally and/or spatially variable second magnetic field by the magnetic actuation system (110) for actuating the shape changeable member (30) for creating a probing movement of the robot (20) at the probing spot (14),
    c) Measuring a quasi-static and/or dynamical shape change of the robot (20) during execution of step b) by an imaging system (120), and
    d) Determining the one or more properties of the material (10) by a data analyzing system (130) by analyzing the quasi-static and/or dynamical shape change measured in step c).

2.  Method according to claim 1,

    wherein for the determination in step d) also the second magnetic field, and/or an expected response of the robot (20) to the magnetic field is considered,
    and/or wherein for the one or more properties one or more members are selected from the group of members comprising adhesion, pH-value, elasticity, stiffness, friction, temperature, liquid wetting and/or viscoelasticity.

3.  Method according to one of the preceding claims,

    wherein for the imaging system (120) one or more members are selected from the group of members comprising ultrasound, X-ray, X-ray fluoroscopy, photo-acoustic imaging, optical coherence tomography and/or optical imaging, and/or wherein the imaging system (120) provides 1 or more, preferably 30 or more, more preferably 100 or more, images of the robot (20) per second,
    and/or wherein the imaging system (120) is used for locating the robot (20) in step a).

4.  Method according to one of the preceding claims,

    wherein in step a) the shape changeable member (30) is actuated for successively creating different types of locomotion of the robot (20), in particular wherein the robot (20) comprises a single shape changeable member (30) formed as a sheet (34), preferably wherein the length (36) of the sheet (34) is at least three times the width (38) of the sheet (34) and the width (38) of the sheet (34) is at least 1 times, in particular at least 10 times, the height (40) of the sheet (34), in particular a sheet (34) of 6.5 mm $\times$ 2 mm $\times$ 0.15 mm,
    and/or wherein in step a) for the type of locomotion of the robot (20) one or more members are selected from the group of members comprising walking, climbing, crawling, rolling, sliding, stick-slipping, surface (12) swimming, and/or submerged swimming,
    and/or wherein the robot (20) comprises two footpads (22) arranged at opposite ends of the shape changeable member (30), and the robot (20) further comprises an adhesive probing patch (50) arranged along the shape changeable member (30) between the two footpads (22).

5.  Method according to claim 4,
    wherein the probing movement comprises a buckling motion of the robot (20), in particular for probing an adhesion and/or pH-value and/or elasticity and/or stiffness and/or friction and/or temperature and/or liquid wetting and/or viscoelasticity of the material (10), wherein as a first sub-step of the buckling motion the footpads (22) are attached to the surface (12) of the material (10) at a distance to each other smaller than their distance along the shape changeable member (30) for forming an arced shape of the robot (20), wherein for a subsequent second sub-step of the buckling motion the probing patch (50) is brought into contact with the surface (12) of the material (10) while the footpads (22) stay attached to the surface (12) of the material (10), and wherein for a subsequent third sub-step of the buckling motion the probing patch (50) is released from the contact with the surface (12) of the material (10) while the footpads (22) stay attached to the surface (12) of the material (10).

6. Method according to claim 5,

wherein the probing patch (50) covers 50% or less, in particular 10% or less, of a length (36) and 10% or more, in particular 90% or more, of a width (38) of the shape changeable member (30), respectively, wherein the probing patch (50) is arranged at the shape changeable member (30) centered between the two footpads (22), in particular wherein the probing patch (50) has dimensions of 0.5 mm × 2 mm × 0.35 mm,
and/or wherein the used probing patch (50) comprises a pH-sensitive and/or protein-sensitive and/or mucus-sensitive and/or glucose-sensitive and/or temperature sensitive adhesion on the material (10) to be probed.

7. Method according to one of the claims 5 or 6,

wherein in the second sub-step the second magnetic field is provided with a direction towards the surface (12) of the material (10), and wherein in the third sub-step the magnetic field is provided with a direction away from the surface (12) of the material (10). in particular wherein in the second sub-step and/or the third sub-step, the second magnetic field is provided perpendicular to the surface (12) of the material (10),
and/or wherein in the second sub-step the second magnetic field is provided with a strength of 1 mT or more for 0.1 seconds or more,
and/or wherein in the third sub-step a strength of the magnetic field is gradually increased over time, in particular uniformly and/or step-wise, preferably wherein in the third sub-step a strength of the magnetic field gradually increased over 0.1 or more seconds, in particular 25 or more seconds, from 0 mT to 10 mT or more, in particular from 0 mT to 500 mT or more, in particular uniformly and/or in steps of 0.1 mT or more.

8. Method according to claim 4,
wherein the probing movement comprises an undulating motion of the robot (20), in particular for probing a viscoelasticity of the material (10), wherein as a first sub-step of the undulating motion the footpads (22) are attached to the surface (12) of the material (10) at a distance to each equal to their distance along the shape changeable member (30) for bringing the robot (20) in contact with the surface (12) of the material (10) along its entire length (36), wherein for a subsequent second sub-step of the undulating motion the shape changeable member (30) is actuated for performing an oscillating motion while maintaining the contact with the surface (12) of the material (10).

9. Method according to claim 8,
wherein the probing patch (50) covers 90% or more of a length (36) and 90% or more of a width (38) of the shape changeable member (30), respectively, wherein the probing patch (50) is arranged at the shape changeable member (30) centered between the two footpads (22), in particular wherein the probing patch (50) has dimensions of 6.5 mm × 2 mm × 0.05 mm, and/or wherein the used probing patch (50) comprises an accordingly selected material (10) which adheres to the material (10) to be probed.

10. Method according to one of the claims 8 or 9,

wherein in first sub-step, the probing patch (50) and hence the robot (20) is pressed against the surface (12) of the material (10) by an accordingly selected second magnetic field with a direction towards the surface (12) of the material (10) for 0.1 seconds or more, in particular for 30 seconds or more, preferably for 60 seconds or more, and/or with a force induced by the first magnetic field of 0.005 mN or more, preferably with a force of 0.1 mN or more,
and/or wherein in the second sub-step the second magnetic field is provided with a constant strength and a changing direction, which is rotated perpendicular to an axis parallel to the width (38) of the shape changeable member (30), in particular wherein the axis is arranged centered between the two footpads (22), in particular wherein the constant strength of the second magnetic field is between 1 mT and 500 mT, in particular between 10 mT and 30 mT, preferably is 20 mT,
and/or wherein a frequency of the rotation of the changing direction is selected between 0.005 Hz and 25 Hz, preferably is 0.1 Hz, in particular for probing a storage modulus of the material (10), in particular wherein for each selected frequency, the measurement of the dynamical shape in step d) is performed with said frequency kept constant for one or more, preferably 10 or more, more preferably 25 or more, complete rotations of the direction of the magnetic field,
and/or wherein the frequency of the rotation of the changing direction is stepwise increased with one steps or more, in particular 5 steps or more, selected within the interval from 0.003 Hz to 150 Hz, in particular for probing a loss modulus of the material (10), wherein the measurement of the dynamical shape in step d) is performed with for each of said frequency steps with the respective frequency kept constant for one or more, preferably 10 or more, more preferably 25 or more, complete rotations of the direction of the magnetic field.

11. Method according to claim 10,
wherein the storage modulus of the material (10) and/or the loss modulus of the material (10) is determined at two or more points, preferably determined continuously, along the length (36) of the shape changeable member (30).

12. Method according to one of the preceding claims 1 to 11,
wherein steps b) to d) are carried out repeatedly, in particular for 5 or more times.

13. Robot for probing one or more properties of a material (10) at a probing spot (14) at the surface (12) of the material (10), wherein the robot (20) is untethered and comprises a shape changeable member (30) actuatable by temporally and/or spatially variable magnetic fields, wherein the robot (20) comprises two footpads (22) arranged at opposite ends of the shape changeable member (30), and the robot (20) further comprises an adhesive probing patch (50) arranged along the shape changeable member (30) between the two footpads (22),
wherein the robot (20) is constructed to be used during execution of the method according to one of the preceding claims.

14. Robot according to claim 13,

wherein the robot (20) comprises only one shape changeable member (30) formed as a sheet (34), wherein the length (36) of the sheet (34) is at least three times the width (38) of the sheet (34) and the width (38) of the sheet (34) is at least 1 times, in particular at least 10 times, the height (40) of the sheet (34), preferably wherein the shape changeable member (30) comprises a thickness-to-length (36) ratio of 0.023, in particular wherein the shape changeable member (30) is a sheet (34) of 6.5 mm × 2 mm × 0.15 mm,
and/or wherein the footpads (22) comprise a hollow-cylinder-shaped body (24), wherein on the body (24) microspikes (26) coated with hydrogel (62) and chitosan-based bio-adhesives (64) are arranged.

15. Robot according to one of the claims 13 or 14,

wherein the adhesive probing patch (50) comprises a three-layer structure, a layer of elastomer (60), preferably with a thickness of 0.1 mm, facing the shape changeable member (30), on top of that a layer of coated hydrogel (62), preferably with a thickness of 0.05 mm, and on top of that a layer of pH-sensitive and/or protein-sensitive and/or mucus-sensitive and/or glucose-sensitive and/or temperature sensitive bio-adhesive (64), preferably with a thickness of 0.2 mm, wherein in particular the probing patch (50) has dimensions of 0.5 mm × 2 mm × 0.35 mm,
or wherein the adhesive probing patch (50) comprises a two-layer structure comprising a hydrogel (62) layer and a layer of chitosan-based bio-adhesives (64), preferably with a total thickness of 0.05 mm, wherein in particular the probing patch (50) has dimensions of 6.5 mm × 2 mm × 0.05 mm.

16. System (100) for probing one or more properties of a material (10) at a probing spot (14) at the surface (12) of the material (10), comprising a magnetic actuation system (110), an imaging system (120), a data analyzing system (130), and a robot (20) according to one of the claims 13 to 15, wherein the system (100) is constructed for carrying out the method according to one of the claims 1 to 12.

**A** Tissue / Mucus / Robot — Top view — Adhesive layer — Adhesive patch — 6.5 mm — 2 mm — Hydrogel — Bio-adhesive

**B** Adhesion strength (N/m²) vs Contact time (1 sec, 30 sec, 1 min, 5 min, 10 min)

**C**
Deployment via climbing
t=0s, gravity, 0.1 mT | t=4.1s, 9.4 mT | t=8.3s, 4.1 mT | t=10.7s, 6.4 mT | t=14.2s, 7.2 mT

Sensing via dynamic actuation
t=25.1s, 7.5mT | t=25.6s, 7.5mT | t=26.3s, 7.5mT | t=27.6s, 7.5mT | t=28.3s, 7.5 mT

Retrieval via climbing
t=30.1s, 26.5 mT | t=36.3s, 28.5 mT | t=43.3s, 28.8 mT | t=48.6s, 28.5 mT | t=51.7s, 7.8 mT

**D** |B| detachment (mT) vs Contact time (1 sec, 30 sec, 1 min, 5 min, 10 min)

**E**
t=0, Air, Agarose gel, 24 mT | t=1.25s, 24 mT | t=2.50s, 24 mT | t=3.75s, 24 mT | t=5.00s, 24 mT | t=6.25s, 24 mT | t=7.50s, 24 mT | t=8.75s, gravity, 24 mT

Fig.5

Fig. 6

Fig. 7

Fig 10

Fig. 11

Fig. 12

Fig. 13

Fig. 15

Fig. 16

**A**

Magnitude

-2000  -1000  0  1000  2000

**B**

**C**

**D**

Fig. 18

Fig. 14

FIG. 20

Fig. 22

**A** $u_y(\mu m)$

$\epsilon_{yy} = \dfrac{\partial u_y}{\partial y}$

**B** $\epsilon_{yy}$

**C** $\overline{\epsilon_{yy}}$

**D**

(i) $u_y^r(s = 0.5L)$

robot $B$

(ii)

$\epsilon_{yy}(x,y) = -\dfrac{2cy^3}{\pi}\displaystyle\int_a^b \dfrac{u_y^r(x')dx'}{[(x-x')^2 + y^2]^2}$

**E** $\epsilon_{yy}/c$ (mm)

**F** $\overline{\epsilon_{yy}}/c$ (mm)

Fig. 23

**A**

Electromagnetic solenoid

Sensed material

Robot

Camera

**B**

Camera

Electromagnetic solenoid

Fig. 24

Fig. 25

EP 4 438 240 A1

Europäisches
Patentamt

European
Patent Office

Office européen
des brevets

# EUROPEAN SEARCH REPORT

Application Number

EP 23 16 5664

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WU YINGDAN ET AL: "Wireless soft millirobots for climbing three-dimensional surfaces in confined spaces", SCIENCE ADVANCES, vol. 8, no. 21, 27 May 2022 (2022-05-27), pages 1-17, XP93078708, DOI: 10.1126/sciadv.abn3431 * the whole document * ----- | 1-16 | INV. B25J9/16 A61B34/30 A61B34/00 B25J11/00 A61M25/01 ADD. A61B34/20 A61B1/00 |
| A | WO 2019/068137 A1 (UNIV WOLLONGONG [AU]) 11 April 2019 (2019-04-11) * the whole document * ----- | 1-16 | |

TECHNICAL FIELDS
SEARCHED        (IPC)

B25J
A61B
A61M

The present search report has been drawn up for all claims

| Place of search | Date of completion of the search | Examiner |
|---|---|---|
| The Hague | 5 September 2023 | De Porcellinis, S |

EPO FORM 1503 03.82 (P04C01)

## ANNEX TO THE EUROPEAN SEARCH REPORT
## ON EUROPEAN PATENT APPLICATION NO.

EP 23 16 5664

This annex lists the patent family members relating to the patent documents cited in the above-mentioned European search report.
The members are as contained in the European Patent Office EDP file on
The European Patent Office is in no way liable for these particulars which are merely given for the purpose of information.

05-09-2023

| Patent document cited in search report | Publication date | Patent family member(s) | Publication date |
|---|---|---|---|
| WO 2019068137 A1 | 11-04-2019 | NONE | |

EPO FORM P0459

For more details about this annex : see Official Journal of the European Patent Office, No. 12/82